(19) Europäisches Patentamt / European Patent Office / Office européen des brevets

(11) EP 2 754 714 A1

(12) # EUROPEAN PATENT APPLICATION

(43) Date of publication:
**16.07.2014 Bulletin 2014/29**

(21) Application number: **13151106.5**

(22) Date of filing: **14.01.2013**

(51) Int Cl.:
***C12N 15/117*** *(2010.01)*
***A61K 31/7088*** *(2006.01)*
*C07H 21/00* *(2006.01)*
*A61P 29/00* *(2006.01)*
*A61P 37/02* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Sarepta Therapeutics, Inc.**
**Cambridge, MA 02142 (US)**

(72) Inventors:
- **Uhlmann, Eugen**
  **61479 Glashütten (DE)**
- **Jurk, Marion**
  **41540 Dormagen (DE)**
- **Lehmann, Thomas**
  **50999 Köln (DE)**

(74) Representative: **Keller, Günter et al**
**Lederer & Keller**
**Patentanwälte Partnerschaft mbB**
**Unsöldstrasse 2**
**80538 München (DE)**

(54) **Inhibitory oligonucleotides and their use in therapy**

(57) Inhibitory oligonucleotide having the general formula:

$$X_1\ C\ C\ N_1\ N_2\ N_3\ X_2\ N_4\ N_5\ G\ G\ G\ N_6\ X_3\ N_7 \qquad (I)$$

are disclosed which can be used in pharmaceutical compositions, whereby in formula (I) C is cytidine or a derivative thereof, whereby the cytidine derivative is selected from the group consisting of 5-methylcytidine, a cytidine-like nucleotide having a chemical modification involving the cytosine base, cytidine nucleoside sugar, or both the cytosine base and the cytidine nucleoside sugar, 2'-O-methylcytidine, 5-bromocytidine, 5-hydroxycytidine, ribocytidine and cytosine-β-D-arabinofuranoside,
G is guanosine or a derivative thereof, whereby the guanosine derivative is selected from the group consisting of 7-deazaguanosine, a guanosine-like nucleotide having a chemical modification involving the guanine base, the guanosine nucleoside sugar or both the guanine base and the guanosine nucleoside sugar,
$X_1$ and $X_3$ is any nucleotide sequence with 0 to 12 bases and each nucleotide is independent of any other, $X_2$ is any nucleotide sequence having 0 to 3 nucleotides,
$N_1$, $N_2$ and $N_3$ are each independently any nucleotide,
$N_4$ and $N_7$ is a pyrimidine or a modified pyrimidine,
$N_5$ is a purin or a modified purin,
$N_6$ is a modified pyrimidine, A or a modified purin,
wherein at least two of the nucleotides $N_4$, $N_5$, $N_6$ or $N_7$ are modified purins or modified pyrimidines.



Printed by Jouve, 75001 PARIS (FR)

SEQUENCE LISTING

<110> Eugen UHLMANN, Marion JURK, Thomas LEHMANN

<120> Inhibitory oligonucleotides and their use in therapy

<130> PEP02253ADI

<160> 199

<170> PatentIn version 3.5

<210> 1
<211> 15
<212> DNA
<213> Artificial Sequence

<220>
<223> Synthetic Oligonucleotide

<400> 1
tcctggcggg gaagt 15

<210> 2
<211> 15
<212> DNA
<213> Artificial Sequence

<220>
<223> Synthetic Oligonucleotide

<220>
<221> modified_base
<222> (8)..(8)
<223> 7-Deaza-dG

<400> 2
tcctggcggg gaagt 15

<210> 3
<211> 15
<212> DNA
<213> Artificial Sequence

<220>
<223> Synthetic Oligonucleotide

<220>
<221> modified_base
<222> (9)..(9)
<223> 7-Deaza-dG

<400> 3
tcctggcggg gaagt 15

<210> 4
<211> 15
<212> DNA
<213> Artificial Sequence

<220>
<223> Synthetic Oligonucleotide

<220>
<221> modified_base
<222> (10)..(10)
<223> 7-Deaza-dG

<400> 4
tcctggcggg gaagt 15

<210> 5
<211> 15
<212> DNA
<213> Artificial Sequence

<220>
<223> Synthetic Oligonucleotide

<220>
<221> modified_base
<222> (11)..(11)
<223> 7-Deaza-dG

<400> 5
tcctggcggg gaagt 15

<210> 6
<211> 15
<212> DNA
<213> Artificial Sequence

<220>
<223> Synthetic Oligonucleotide

<220>
<221> modified_base
<222> (8)..(8)
<223> 7-Deaza-2'-O-methyl-G

<400> 6
tcctggcggg gaagt 15

<210> 7
<211> 15
<212> DNA
<213> Artificial Sequence

<220>
<223> Synthetic Oligonucleotide

<220>
<221> modified_base
<222> (9)..(9)
<223> 7-Deaza-2'-O-methyl-G

<400> 7
tcctggcggg gaagt 15

<210> 8
<211> 15
<212> DNA
<213> Artificial Sequence

<220>
<223> Synthetic Oligonucleotide

<220>
<221> modified_base
<222> (10)..(10)
<223> 7-Deaza-2'-O-methyl-G

<400> 8
tcctggcggg gaagt 15

<210> 9
<211> 15
<212> DNA
<213> Artificial Sequence

<220>
<223> Synthetic Oligonucleotide

<220>
<221> modified_base
<222> (11)..(11)
<223> 7-Deaza-2'-O-methyl-G

<400> 9
tcctggcggg gaagt 15

<210> 10
<211> 15
<212> DNA
<213> Artificial Sequence

<220>
<223> Synthetic Oligonucleotide

<220>
<221> modified_base
<222> (8)..(8)
<223> I

<400> 10
tcctggcggg gaagt 15

<210> 11
<211> 15
<212> DNA
<213> Artificial Sequence

<220>
<223> Synthetic Oligonucleotide

<220>
<221> modified_base
<222> (9)..(9)
<223> I

<400> 11
tcctggcggg gaagt 15

<210> 12
<211> 15
<212> DNA
<213> Artificial Sequence

<220>
<223> Synthetic Oligonucleotide

<220>
<221> modified_base
<222> (10)..(10)
<223> I

<400> 12
tcctggcggg gaagt 15

<210> 13
<211> 15
<212> DNA
<213> Artificial Sequence

<220>
<223> Synthetic Oligonucleotide

<220>
<221> modified_base
<222> (11)..(11)
<223> I

<400> 13
tcctggcggg gaagt 15

<210> 14
<211> 15
<212> DNA
<213> Artificial Sequence

<220>
<223> Synthetic Oligonucleotide

<220>
<221> modified_base
<222> (8)..(8)
<223> 2,6-Diaminopurine

<400> 14
tcctggcagg gaagt 15

<210> 15
<211> 15

<212> DNA
<213> Artificial Sequence

<220>
<223> Synthetic Oligonucleotide

<220>
<221> modified_base
<222> (9)..(9)
<223> 2,6-Diaminopurine

<400> 15
tcctggcgag gaagt 15

<210> 16
<211> 15
<212> DNA
<213> Artificial Sequence

<220>
<223> Synthetic Oligonucleotide

<220>
<221> modified_base
<222> (10)..(10)
<223> 2,6-Diaminopurine

<400> 16
tcctggcgga gaagt 15

<210> 17
<211> 15
<212> DNA
<213> Artificial Sequence

<220>
<223> Synthetic Oligonucleotide

<220>
<221> modified_base
<222> (11)..(11)
<223> 2,6-Diaminopurine

<400> 17
tcctggcggg aaagt 15

<210> 18
<211> 15
<212> DNA
<213> Artificial Sequence

<220>
<223> Synthetic Oligonucleotide

<220>
<221> modified_base
<222> (8)..(8)
<223> 7-Deaza-8-aza-dG

<400> 18
tcctggcggg gaagt 15

<210> 19
<211> 15
<212> DNA
<213> Artificial Sequence

<220>
<223> Synthetic Oligonucleotide

<220>
<221> modified_base
<222> (9)..(9)
<223> 7-Deaza-8-aza-dG

<400> 19
tcctggcggg gaagt 15

<210> 20
<211> 15
<212> DNA
<213> Artificial Sequence

<220>
<223> Synthetic Oligonucleotide

<220>
<221> modified_base
<222> (10)..(10)
<223> 7-Deaza-8-aza-dG

<400> 20
tcctggcggg gaagt 15

<210> 21
<211> 15
<212> DNA
<213> Artificial Sequence

<220>
<223> Synthetic Oligonucleotide

<220>
<221> modified_base
<222> (11)..(11)
<223> 7-Deaza-8-aza-dG

<400> 21
tcctggcggg gaagt 15

<210> 22
<211> 15
<212> DNA
<213> Artificial Sequence

<220>

<223> Synthetic Oligonucleotide

<220>
<221> modified_base
<222> (8)..(8)
<223> 8-Oxo-dG

<400> 22
tcctggcggg gaagt 15

<210> 23
<211> 15
<212> DNA
<213> Artificial Sequence

<220>
<223> Synthetic Oligonucleotide

<220>
<221> modified_base
<222> (10)..(10)
<223> 8-Oxo-dG

<400> 23
tcctggcggg gaagt 15

<210> 24
<211> 15
<212> DNA
<213> Artificial Sequence

<220>
<223> Synthetic Oligonucleotide

<220>
<221> modified_base
<222> (11)..(11)
<223> 8-Oxo-dG

<400> 24
tcctggcggg gaagt 15

<210> 25
<211> 15
<212> DNA
<213> Artificial Sequence

<220>
<223> Synthetic Oligonucleotide

<220>
<221> modified_base
<222> (8)..(8)
<223> 8-Oxo-dA

<400> 25
tcctggcagg gaagt 15

<210> 26
<211> 15
<212> DNA
<213> Artificial Sequence

<220>
<223> Synthetic Oligonucleotide

<220>
<221> modified_base
<222> (9)..(9)
<223> 8-Oxo-dA

<400> 26
tcctggcgag gaagt 15

<210> 27
<211> 15
<212> DNA
<213> Artificial Sequence

<220>
<223> Synthetic Oligonucleotide

<220>
<221> modified_base
<222> (10)..(10)
<223> 8-Oxo-dA

<400> 27
tcctggcgga gaagt 15

<210> 28
<211> 15
<212> DNA
<213> Artificial Sequence

<220>
<223> Synthetic Oligonucleotide

<220>
<221> modified_base
<222> (11)..(11)
<223> 8-Oxo-dA

<400> 28
tcctggcggg aaagt 15

<210> 29
<211> 15
<212> DNA
<213> Artificial Sequence

<220>
<223> Synthetic Oligonucleotide

<220>

<221> modified_base
<222> (8)..(8)
<223> 6-Thio-dG

<400> 29
tcctggcggg gaagt 15

<210> 30
<211> 15
<212> DNA
<213> Artificial Sequence

<220>
<223> Synthetic Oligonucleotide

<220>
<221> modified_base
<222> (9)..(9)
<223> 6-Thio-dG

<400> 30
tcctggcggg gaagt 15

<210> 31
<211> 15
<212> DNA
<213> Artificial Sequence

<220>
<223> Synthetic Oligonucleotide

<220>
<221> modified_base
<222> (10)..(10)
<223> 6-Thio-dG

<400> 31
tcctggcggg gaagt 15

<210> 32
<211> 15
<212> DNA
<213> Artificial Sequence

<220>
<223> Synthetic Oligonucleotide

<220>
<221> modified_base
<222> (11)..(11)
<223> 6-Thio-dG

<400> 32
tcctggcggg gaagt 15

<210> 33
<211> 15
<212> DNA

<213> Artificial Sequence

<220>
<223> Synthetic Oligonucleotide

<220>
<221> modified_base
<222> (8)..(8)
<223> 6-O-methyl-dG

<400> 33
tcctggcggg gaagt 15

<210> 34
<211> 15
<212> DNA
<213> Artificial Sequence

<220>
<223> Synthetic Oligonucleotide

<220>
<221> modified_base
<222> (9)..(9)
<223> 6-O-methyl-dG

<400> 34
tcctggcggg gaagt 15

<210> 35
<211> 15
<212> DNA
<213> Artificial Sequence

<220>
<223> Synthetic Oligonucleotide

<220>
<221> modified_base
<222> (11)..(11)
<223> 6-O-methyl-dG

<400> 35
tcctggcggg gaagt 15

<210> 36
<211> 15
<212> DNA
<213> Artificial Sequence

<220>
<223> Synthetic Oligonucleotide

<220>
<221> modified_base
<222> (8)..(8)
<223> 7-Deazainosine

<400> 36
tcctggcggg gaagt 15

<210> 37
<211> 15
<212> DNA
<213> Artificial Sequence

<220>
<223> Synthetic Oligonucleotide

<220>
<221> modified_base
<222> (9)..(9)
<223> 7-Deazainosine

<400> 37
tcctggcggg gaagt 15

<210> 38
<211> 15
<212> DNA
<213> Artificial Sequence

<220>
<223> Synthetic Oligonucleotide

<220>
<221> modified_base
<222> (10)..(10)
<223> 7-Deazainosine

<400> 38
tcctggcggg gaagt 15

<210> 39
<211> 15
<212> DNA
<213> Artificial Sequence

<220>
<223> Synthetic Oligonucleotide

<220>
<221> modified_base
<222> (11)..(11)
<223> 7-Deazainosine

<400> 39
tcctggcggg gaagt 15

<210> 40
<211> 15
<212> DNA
<213> Artificial Sequence

<220>
<223> Synthetic Oligonucleotide

<220>
<221> modified_base
<222> (8)..(8)
<223> 7-Deaza-7-iodo-dG

<400> 40
tcctggcggg gaagt 15

<210> 41
<211> 15
<212> DNA
<213> Artificial Sequence

<220>
<223> Synthetic Oligonucleotide

<220>
<221> modified_base
<222> (9)..(9)
<223> 7-Deaza-7-iodo-dG

<400> 41
tcctggcggg gaagt 15

<210> 42
<211> 15
<212> DNA
<213> Artificial Sequence

<220>
<223> Synthetic Oligonucleotide

<220>
<221> modified_base
<222> (10)..(10)
<223> 7-Deaza-7-iodo-dG

<400> 42
tcctggcggg gaagt 15

<210> 43
<211> 15
<212> DNA
<213> Artificial Sequence

<220>
<223> Synthetic Oligonucleotide

<220>
<221> modified_base
<222> (11)..(11)
<223> 7-Deaza-7-iodo-dG

<400> 43
tcctggcggg gaagt 15

<210> 44
<211> 15
<212> DNA
<213> Artificial Sequence

<220>
<223> Synthetic Oligonucleotide

<220>
<221> modified_base
<222> (8)..(8)
<223> 7-Aminopropargyl-dG

<400> 44
tcctggcggg gaagt 15

<210> 45
<211> 15
<212> DNA
<213> Artificial Sequence

<220>
<223> Synthetic Oligonucleotide

<220>
<221> modified_base
<222> (9)..(9)
<223> 7-Aminopropargyl-dG

<400> 45
tcctggcggg gaagt 15

<210> 46
<211> 15
<212> DNA
<213> Artificial Sequence

<220>
<223> Synthetic Oligonucleotide

<220>
<221> modified_base
<222> (10)..(10)
<223> 7-Aminopropargyl-dG

<400> 46
tcctggcggg gaagt 15

<210> 47
<211> 15
<212> DNA
<213> Artificial Sequence

<220>
<223> Synthetic Oligonucleotide

<220>
<221> modified_base

<222>  (11)..(11)
<223>  7-Aminopropargyl-dG

<400>  47
tcctggcggg gaagt    15

<210>  48
<211>  15
<212>  DNA
<213>  Artificial Sequence

<220>
<223>  Synthetic Oligonucleotide

<400>  48
tcctggcagg gaagt    15

<210>  49
<211>  15
<212>  DNA
<213>  Artificial Sequence

<220>
<223>  Synthetic Oligonucleotide

<400>  49
tcctggcgga gaagt    15

<210>  50
<211>  15
<212>  DNA
<213>  Artificial Sequence

<220>
<223>  Synthetic Oligonucleotide

<400>  50
tcctggcggg aaagt    15

<210>  51
<211>  15
<212>  DNA
<213>  Artificial Sequence

<220>
<223>  Synthetic Oligonucleotide

<400>  51
tcctggcgga gaggt    15

<210>  52
<211>  15
<212>  DNA
<213>  Artificial Sequence

<220>
<223>  Synthetic Oligonucleotide

<400>  52
tcctggcggg aaggt    15

<210> 53
<211> 15
<212> DNA
<213> Artificial Sequence

<220>
<223> Synthetic Oligonucleotide

<400> 53
tcctggctgg gaagt 15

<210> 54
<211> 15
<212> DNA
<213> Artificial Sequence

<220>
<223> Synthetic Oligonucleotide

<400> 54
tcctggcgtg gaagt 15

<210> 55
<211> 15
<212> DNA
<213> Artificial Sequence

<220>
<223> Synthetic Oligonucleotide

<400> 55
tcctggcggt gaagt 15

<210> 56
<211> 15
<212> DNA
<213> Artificial Sequence

<220>
<223> Synthetic Oligonucleotide

<400> 56
tcctggcggg taagt 15

<210> 57
<211> 15
<212> DNA
<213> Artificial Sequence

<220>
<223> Synthetic Oligonucleotide

<220>
<221> modified_base
<222> (8)..(8)
<223> 5-Methyl-dC

<400> 57

tcctggccgg gaagt 15

<210> 58
<211> 15
<212> DNA
<213> Artificial Sequence

<220>
<223> Synthetic Oligonucleotide

<220>
<221> modified_base
<222> (9)..(9)
<223> 5-Methyl-dC

<400> 58
tcctggcgcg gaagt 15

<210> 59
<211> 15
<212> DNA
<213> Artificial Sequence

<220>
<223> Synthetic Oligonucleotide

<220>
<221> modified_base
<222> (10)..(10)
<223> 5-Methyl-dC

<400> 59
tcctggcggc gaagt 15

<210> 60
<211> 15
<212> DNA
<213> Artificial Sequence

<220>
<223> Synthetic Oligonucleotide

<220>
<221> modified_base
<222> (11)..(11)
<223> 5-Methyl-dC

<400> 60
tcctggcggg caagt 15

<210> 61
<211> 15
<212> DNA
<213> Artificial Sequence

<220>
<223> Synthetic Oligonucleotide

<400> 61
tcctggcugg gaagt 15

<210> 62
<211> 15
<212> DNA
<213> Artificial Sequence

<220>
<223> Synthetic Oligonucleotide

<400> 62
tcctggcgug gaagt 15

<210> 63
<211> 15
<212> DNA
<213> Artificial Sequence

<220>
<223> Synthetic Oligonucleotide

<400> 63
tcctggcggu gaagt 15

<210> 64
<211> 15
<212> DNA
<213> Artificial Sequence

<220>
<223> Synthetic Oligonucleotide

<400> 64
tcctggcggg uaagt 15

<210> 65
<211> 15
<212> DNA
<213> Artificial Sequence

<220>
<223> Synthetic Oligonucleotide

<220>
<221> misc_feature
<222> (8)..(8)
<223> dSpacer, abasic

<400> 65
tcctggcngg gaagt 15

<210> 66
<211> 15
<212> DNA
<213> Artificial Sequence

<220>
<223> Synthetic Oligonucleotide

<220>
<221> misc_feature
<222> (9)..(9)
<223> dSpacer, abasic

<400> 66
tcctggcgng gaagt 15

<210> 67
<211> 15
<212> DNA
<213> Artificial Sequence

<220>
<223> Synthetic Oligonucleotide

<220>
<221> misc_feature
<222> (10)..(10)
<223> dSpacer, abasic

<400> 67
tcctggcggn gaagt 15

<210> 68
<211> 15
<212> DNA
<213> Artificial Sequence

<220>
<223> Synthetic Oligonucleotide

<220>
<221> misc_feature
<222> (11)..(11)
<223> dSpacer, abasic

<400> 68
tcctggcggg naagt 15

<210> 69
<211> 15
<212> DNA
<213> Artificial Sequence

<220>
<223> Synthetic Oligonucleotide

<220>
<221> misc_feature
<222> (8)..(8)
<223> C3 spacer

<400> 69
tcctggcngg gaagt 15

<210> 70
<211> 15
<212> DNA
<213> Artificial Sequence

<220>
<223> Synthetic Oligonucleotide

<220>
<221> misc_feature
<222> (9)..(9)
<223> C3 spacer

<400> 70
tcctggcgng gaagt 15

<210> 71
<211> 15
<212> DNA
<213> Artificial Sequence

<220>
<223> Synthetic Oligonucleotide

<220>
<221> misc_feature
<222> (10)..(10)
<223> C3 spacer

<400> 71
tcctggcggn gaagt 15

<210> 72
<211> 15
<212> DNA
<213> Artificial Sequence

<220>
<223> Synthetic Oligonucleotide

<220>
<221> misc_feature
<222> (11)..(11)
<223> C3 spacer

<400> 72
tcctggcggg naagt 15

<210> 73
<211> 16
<212> DNA
<213> Artificial Sequence

<220>
<223> Synthetic Oligonucleotide

<220>
<221> misc_feature

<222> (9)..(9)
<223> dSpacer, abasic

<400> 73
tcctggcgng ggaagt 16

<210> 74
<211> 16
<212> DNA
<213> Artificial Sequence

<220>
<223> Synthetic Oligonucleotide

<220>
<221> misc_feature
<222> (10)..(10)
<223> dSpacer, abasic

<400> 74
tcctggcggn ggaagt 16

<210> 75
<211> 16
<212> DNA
<213> Artificial Sequence

<220>
<223> Synthetic Oligonucleotide

<220>
<221> misc_feature
<222> (11)..(11)
<223> dSpacer, abasic

<400> 75
tcctggcggg ngaagt 16

<210> 76
<211> 16
<212> DNA
<213> Artificial Sequence

<220>
<223> Synthetic Oligonucleotide

<220>
<221> misc_feature
<222> (9)..(9)
<223> C3 spacer

<400> 76
tcctggcgng ggaagt 16

<210> 77
<211> 16
<212> DNA
<213> Artificial Sequence

<220>
<223> Synthetic Oligonucleotide

<220>
<221> misc_feature
<222> (10)..(10)
<223> C3 spacer

<400> 77
tcctggcggn ggaagt 16

<210> 78
<211> 16
<212> DNA
<213> Artificial Sequence

<220>
<223> Synthetic Oligonucleotide

<220>
<221> misc_feature
<222> (11)..(11)
<223> C3 spacer

<400> 78
tcctggcggg ngaagt 16

<210> 79
<211> 16
<212> DNA
<213> Artificial Sequence

<220>
<223> Synthetic Oligonucleotide

<400> 79
tcctggcgag ggaagt 16

<210> 80
<211> 16
<212> DNA
<213> Artificial Sequence

<220>
<223> Synthetic Oligonucleotide

<400> 80
tcctggcgga ggaagt 16

<210> 81
<211> 16
<212> DNA
<213> Artificial Sequence

<220>
<223> Synthetic Oligonucleotide

<400> 81

tcctggcggg agaagt 16

<210> 82
<211> 16
<212> DNA
<213> Artificial Sequence

<220>
<223> Synthetic Oligonucleotide

<220>
<221> modified_base
<222> (9)..(9)
<223> 7-Deaza-dG

<400> 82
tcctggcggg ggaagt 16

<210> 83
<211> 16
<212> DNA
<213> Artificial Sequence

<220>
<223> Synthetic Oligonucleotide

<220>
<221> modified_base
<222> (10)..(10)
<223> 7-Deaza-dG

<400> 83
tcctggcggg ggaagt 16

<210> 84
<211> 16
<212> DNA
<213> Artificial Sequence

<220>
<223> Synthetic Oligonucleotide

<220>
<221> modified_base
<222> (11)..(11)
<223> 7-Deaza-dG

<400> 84
tcctggcggg ggaagt 16

<210> 85
<211> 15
<212> DNA
<213> Artificial Sequence

<220>
<223> Synthetic Oligonucleotide

<220>
<221> modified_base
<222> (1)..(1)
<223> 5-Iododeoxyuridine

<400> 85
ucctggcggg gaagt 15

<210> 86
<211> 15
<212> DNA
<213> Artificial Sequence

<220>
<223> Synthetic Oligonucleotide

<220>
<221> modified_base
<222> (4)..(4)
<223> 5-Iododeoxyuridine

<400> 86
tccuggcggg gaagt 15

<210> 87
<211> 15
<212> DNA
<213> Artificial Sequence

<220>
<223> Synthetic Oligonucleotide

<220>
<221> modified_base
<222> (1)..(1)
<223> 5-Iododeoxyuridine

<220>
<221> modified_base
<222> (4)..(4)
<223> 5-Iododeoxyuridine

<400> 87
uccuggcggg gaagt 15

<210> 88
<211> 15
<212> DNA
<213> Artificial Sequence

<220>
<223> Synthetic Oligonucleotide

<220>
<221> modified_base
<222> (15)..(15)
<223> 5-Iododeoxyuridine

<400> 88
tcctggcggg gaagu 15

<210> 89
<211> 15
<212> DNA
<213> Artificial Sequence

<220>
<223> Synthetic Oligonucleotide

<220>
<221> modified_base
<222> (7)..(7)
<223> 5-Iododeoxyuridine

<400> 89
tcctgguggg gaagt 15

<210> 90
<211> 15
<212> DNA
<213> Artificial Sequence

<220>
<223> Synthetic Oligonucleotide

<220>
<221> modified_base
<222> (2)..(2)
<223> 2'-Fluorocytosine

<400> 90
tcctggcggg gaagt 15

<210> 91
<211> 15
<212> DNA
<213> Artificial Sequence

<220>
<223> Synthetic Oligonucleotide

<220>
<221> modified_base
<222> (3)..(3)
<223> 2'-Fluorocytosine

<400> 91
tcctggcggg gaagt 15

<210> 92
<211> 15
<212> DNA
<213> Artificial Sequence

<220>
<223> Synthetic Oligonucleotide

<220>
<221> modified_base
<222> (2)..(2)
<223> 2'-Fluorocytosine

<220>
<221> modified_base
<222> (3)..(3)
<223> 2'-Fluorocytosine

<400> 92
tcctggcggg gaagt 15

<210> 93
<211> 15
<212> DNA
<213> Artificial Sequence

<220>
<223> Synthetic Oligonucleotide

<220>
<221> modified_base
<222> (7)..(7)
<223> 2'-Fluorocytosine

<400> 93
tcctggcggg gaagt 15

<210> 94
<211> 15
<212> DNA
<213> Artificial Sequence

<220>
<223> Synthetic Oligonucleotide

<220>
<221> modified_base
<222> (2)..(2)
<223> 2'-FANA-C

<400> 94
tcctggcggg gaagt 15

<210> 95
<211> 15
<212> DNA
<213> Artificial Sequence

<220>
<223> Synthetic Oligonucleotide

<220>
<221> modified_base
<222> (3)..(3)
<223> 2'-FANA-C

<400> 95
tcctggcggg gaagt 15

<210> 96
<211> 15
<212> DNA
<213> Artificial Sequence

<220>
<223> Synthetic Oligonucleotide

<220>
<221> modified_base
<222> (2)..(2)
<223> 2'-FANA-C

<220>
<221> modified_base
<222> (3)..(3)
<223> 2'-FANA-C

<400> 96
tcctggcggg gaagt 15

<210> 97
<211> 15
<212> DNA
<213> Artificial Sequence

<220>
<223> Synthetic Oligonucleotide

<220>
<221> modified_base
<222> (7)..(7)
<223> 2'-FANA-C

<400> 97
tcctggcggg gaagt 15

<210> 98
<211> 15
<212> DNA
<213> Artificial Sequence

<220>
<223> Synthetic Oligonucleotide

<220>
<221> modified_base
<222> (12)..(12)
<223> 3' Adenine as 2'-O-methyl

<220>
<221> modified_base
<222> (13)..(13)
<223> 3' Adenine as 2'-O-methyl

<220>
<221> modified_base
<222> (14)..(14)
<223> 3′ Guanine as 2′-O-methyl

<400> 98
tcctggcggg gaagt 15

<210> 99
<211> 15
<212> DNA
<213> Artificial Sequence

<220>
<223> Synthetic Oligonucleotide

<220>
<221> modified_base
<222> (12)..(12)
<223> 3′ Adenine as 2′-F

<220>
<221> modified_base
<222> (13)..(13)
<223> 3′ Adenine as 2′-F

<220>
<221> modified_base
<222> (14)..(14)
<223> 3′ Guanine as 2′-F

<400> 99
tcctggcggg gaagt 15

<210> 100
<211> 15
<212> DNA
<213> Artificial Sequence

<220>
<223> Synthetic Oligonucleotide

<220>
<221> modified_base
<222> (12)..(12)
<223> 3′ Adenine as LNA

<220>
<221> modified_base
<222> (13)..(13)
<223> 3′ Adenine as LNA

<220>
<221> modified_base
<222> (14)..(14)
<223> 3′ Guanine as LNA

<400> 100
tcctggcggg gaagt 15

<210> 101
<211> 15
<212> DNA
<213> Artificial Sequence

<220>
<223> Synthetic Oligonucleotide

<220>
<221> modified_base
<222> (7)..(7)
<223> 5-Methyl-dC

<400> 101
tcctggcggg gaagt 15

<210> 102
<211> 15
<212> DNA
<213> Artificial Sequence

<220>
<223> Synthetic Oligonucleotide

<400> 102
tcctggaggg gaagt 15

<210> 103
<211> 15
<212> DNA
<213> Artificial Sequence

<220>
<223> Synthetic Oligonucleotide

<400> 103
tcctggtggg gaagt 15

<210> 104
<211> 15
<212> DNA
<213> Artificial Sequence

<220>
<223> Synthetic Oligonucleotide

<400> 104
tcctgguggg gaagt 15

<210> 105
<211> 15
<212> DNA
<213> Artificial Sequence

<220>
<223> Synthetic Oligonucleotide

<220>
<221> misc_feature

<222> (7)..(7)
<223> dSpacer, abasic

<400> 105
tcctggnggg gaagt 15

<210> 106
<211> 15
<212> DNA
<213> Artificial Sequence

<220>
<223> Synthetic Oligonucleotide

<220>
<221> modified_base
<222> (7)..(7)
<223> 7-Deaza-dG

<400> 106
tcctgggggg gaagt 15

<210> 107
<211> 15
<212> DNA
<213> Artificial Sequence

<220>
<223> Synthetic Oligonucleotide

<220>
<221> modified_base
<222> (7)..(7)
<223> 7-Deaza-dG

<220>
<221> modified_base
<222> (9)..(9)
<223> 7-Deaza-dG

<400> 107
tcctgggggg gaagt 15

<210> 108
<211> 15
<212> DNA
<213> Artificial Sequence

<220>
<223> Synthetic Oligonucleotide

<220>
<221> modified_base
<222> (7)..(7)
<223> 7-Deaza-dG

<400> 108
tcctggggag gaagt 15

<210> 109
<211> 15
<212> DNA
<213> Artificial Sequence

<220>
<223> Synthetic Oligonucleotide

<220>
<221> modified_base
<222> (7)..(7)
<223> cm

<400> 109
tcctggcggg gaagt 15

<210> 110
<211> 15
<212> DNA
<213> Artificial Sequence

<220>
<223> Synthetic Oligonucleotide

<220>
<221> modified_base
<222> (7)..(7)
<223> LNA-5-methyl-C

<400> 110
tcctggcggg gaagt 15

<210> 111
<211> 10
<212> DNA
<213> Artificial Sequence

<220>
<223> Synthetic Oligonucleotide

<400> 111
cctggcgggg 10

<210> 112
<211> 10
<212> DNA
<213> Artificial Sequence

<220>
<223> Synthetic Oligonucleotide

<220>
<221> modified_base
<222> (7)..(7)
<223> 7-Deaza-dG

<400> 112
cctggcgggg 10

<210> 113
<211> 10
<212> DNA
<213> Artificial Sequence

<220>
<223> Synthetic Oligonucleotide

<220>
<221> modified_base
<222> (8)..(8)
<223> 7-Deaza-dG

<400> 113
cctggcgggg 10

<210> 114
<211> 10
<212> DNA
<213> Artificial Sequence

<220>
<223> Synthetic Oligonucleotide

<220>
<221> modified_base
<222> (9)..(9)
<223> 7-Deaza-dG

<400> 114
cctggcgggg 10

<210> 115
<211> 10
<212> DNA
<213> Artificial Sequence

<220>
<223> Synthetic Oligonucleotide

<220>
<221> modified_base
<222> (10)..(10)
<223> 7-Deaza-dG

<400> 115
cctggcgggg 10

<210> 116
<211> 10
<212> DNA
<213> Artificial Sequence

<220>
<223> Synthetic Oligonucleotide

<220>
<221> modified_base
<222> (7)..(7)
<223> 7-Deaza-2'-O-methyl-G

<400> 116
cctggcgggg 10

<210> 117
<211> 10
<212> DNA
<213> Artificial Sequence

<220>
<223> Synthetic Oligonucleotide

<220>
<221> modified_base
<222> (8)..(8)
<223> 7-Deaza-2'-O-methyl-G

<400> 117
cctggcgggg 10

<210> 118
<211> 10
<212> DNA
<213> Artificial Sequence

<220>
<223> Synthetic Oligonucleotide

<220>
<221> modified_base
<222> (9)..(9)
<223> 7-Deaza-2'-O-methyl-G

<400> 118
cctggcgggg 10

<210> 119
<211> 10
<212> DNA
<213> Artificial Sequence

<220>
<223> Synthetic Oligonucleotide

<220>
<221> modified_base
<222> (10)..(10)
<223> 7-Deaza-2'-O-methyl-G

<400> 119
cctggcgggg 10

<210> 120
<211> 10

<212> DNA
<213> Artificial Sequence

<220>
<223> Synthetic Oligonucleotide

<220>
<221> modified_base
<222> (8)..(8)
<223> I

<400> 120
cctggcgggg 10

<210> 121
<211> 10
<212> DNA
<213> Artificial Sequence

<220>
<223> Synthetic Oligonucleotide

<220>
<221> modified_base
<222> (9)..(9)
<223> I

<400> 121
cctggcgggg 10

<210> 122
<211> 10
<212> DNA
<213> Artificial Sequence

<220>
<223> Synthetic Oligonucleotide

<220>
<221> modified_base
<222> (10)..(10)
<223> I

<400> 122
cctggcgggg 10

<210> 123
<211> 10
<212> DNA
<213> Artificial Sequence

<220>
<223> Synthetic Oligonucleotide

<220>
<221> modified_base
<222> (8)..(8)
<223> 6-Thio-dG

<400> 123
cctggcgggg 10

<210> 124
<211> 10
<212> DNA
<213> Artificial Sequence

<220>
<223> Synthetic Oligonucleotide

<220>
<221> modified_base
<222> (9)..(9)
<223> 6-Thio-dG

<400> 124
cctggcgggg 10

<210> 125
<211> 10
<212> DNA
<213> Artificial Sequence

<220>
<223> Synthetic Oligonucleotide

<220>
<221> modified_base
<222> (10)..(10)
<223> 6-Thio-dG

<400> 125
cctggcgggg 10

<210> 126
<211> 10
<212> DNA
<213> Artificial Sequence

<220>
<223> Synthetic Oligonucleotide

<220>
<221> modified_base
<222> (8)..(8)
<223> 7-Deazainosine

<400> 126
cctggcgggg 10

<210> 127
<211> 10
<212> DNA
<213> Artificial Sequence

<220>

<223> Synthetic Oligonucleotide

<220>
<221> modified_base
<222> (9)..(9)
<223> 7-Deazainosine

<400> 127
cctggcgggg 10

<210> 128
<211> 10
<212> DNA
<213> Artificial Sequence

<220>
<223> Synthetic Oligonucleotide

<220>
<221> modified_base
<222> (10)..(10)
<223> 7-Deazainosine

<400> 128
cctggcgggg 10

<210> 129
<211> 10
<212> DNA
<213> Artificial Sequence

<220>
<223> Synthetic Oligonucleotide

<400> 129
cctggcgtgg 10

<210> 130
<211> 10
<212> DNA
<213> Artificial Sequence

<220>
<223> Synthetic Oligonucleotide

<400> 130
cctggcggtg 10

<210> 131
<211> 10
<212> DNA
<213> Artificial Sequence

<220>
<223> Synthetic Oligonucleotide

<400> 131
cctggcgggt 10

<210> 132
<211> 10
<212> DNA
<213> Artificial Sequence

<220>
<223> Synthetic Oligonucleotide

<220>
<221> modified_base
<222> (7)..(7)
<223> 5-Methyl-dC

<400> 132
cctggccggg 10

<210> 133
<211> 10
<212> DNA
<213> Artificial Sequence

<220>
<223> Synthetic Oligonucleotide

<220>
<221> modified_base
<222> (8)..(8)
<223> 5-Methyl-dC

<400> 133
cctggcgcgg 10

<210> 134
<211> 10
<212> DNA
<213> Artificial Sequence

<220>
<223> Synthetic Oligonucleotide

<220>
<221> modified_base
<222> (9)..(9)
<223> 5-Methyl-dC

<400> 134
cctggcggcg 10

<210> 135
<211> 10
<212> DNA
<213> Artificial Sequence

<220>
<223> Synthetic Oligonucleotide

<220>

<221> modified_base
<222> (10)..(10)
<223> 5-Methyl-dC

<400> 135
cctggcgggc 10

<210> 136
<211> 10
<212> DNA
<213> Artificial Sequence

<220>
<223> Synthetic Oligonucleotide

<400> 136
cctggcuggg 10

<210> 137
<211> 10
<212> DNA
<213> Artificial Sequence

<220>
<223> Synthetic Oligonucleotide

<400> 137
cctggcgugg 10

<210> 138
<211> 10
<212> DNA
<213> Artificial Sequence

<220>
<223> Synthetic Oligonucleotide

<400> 138
cctggcggug 10

<210> 139
<211> 10
<212> DNA
<213> Artificial Sequence

<220>
<223> Synthetic Oligonucleotide

<400> 139
cctggcgggu 10

<210> 140
<211> 15
<212> DNA
<213> Artificial Sequence

<220>
<223> Synthetic Oligonucleotide

<220>
<221> modified_base
<222> (7)..(7)
<223> 5-Methyl-dC

<220>
<221> modified_base
<222> (8)..(8)
<223> 7-Deaza-dG

<400> 140
tcctggcggg gaagt 15

<210> 141
<211> 15
<212> DNA
<213> Artificial Sequence

<220>
<223> Synthetic Oligonucleotide

<220>
<221> modified_base
<222> (7)..(7)
<223> 5-Methyl-dC

<220>
<221> modified_base
<222> (8)..(8)
<223> 7-Deaza-2'-O-methyl-G

<400> 141
tcctggcggg gaagt 15

<210> 142
<211> 15
<212> DNA
<213> Artificial Sequence

<220>
<223> Synthetic Oligonucleotide

<220>
<221> modified_base
<222> (7)..(7)
<223> 5-Methyl-dC

<220>
<221> modified_base
<222> (8)..(8)
<223> I

<400> 142
tcctggcggg gaagt 15

<210> 143
<211> 15
<212> DNA
<213> Artificial Sequence

<220>
<223> Synthetic Oligonucleotide

<220>
<221> modified_base
<222> (7)..(7)
<223> 5-Methyl-dC

<220>
<221> modified_base
<222> (8)..(8)
<223> 7-Deazainosine

<400> 143
tcctggcggg gaagt 15

<210> 144
<211> 15
<212> DNA
<213> Artificial Sequence

<220>
<223> Synthetic Oligonucleotide

<220>
<221> modified_base
<222> (7)..(7)
<223> 5-Methyl-dC

<400> 144
tcctggctgg gaagt 15

<210> 145
<211> 10
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deaza-dG

<400> 145
cctggcgggg 10

<210> 146
<211> 10
<212> DNA
<213> Artificial Sequence

<220>

<223> Synthetic Oligonucleotide

<220>
<221> modified_base
<222> (6)..(6)
<223> 5-Methyl-dC

<220>
<221> modified_base
<222> (7)..(7)
<223> 7-Deaza-2'-O-methyl-G

<400> 146
cctggcgggg 10

<210> 147
<211> 10
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deazainosine

<400> 147
cctggcgggg 10

<210> 148
<211> 10
<212> DNA
<213> Artificial Sequence

<220>
<223> Synthetic Oligonucleotide

<220>
<221> modified_base
<222> (6)..(6)
<223> 5-Methyl-dC

<400> 148
cctggctggg 10

<210> 149
<211> 15
<212> DNA
<213> Artificial Sequence

<220>
<223> Synthetic Oligonucleotide

<220>
<221> modified_base
<222> (8)..(8)
<223> 7-Deaza-dG

<220>
<221> modified_base
<222> (12)..(12)
<223> 3' Adenine as 2'-O-methyl

<220>
<221> modified_base
<222> (13)..(13)
<223> 3' Adenine as 2'-O-methyl

<220>
<221> modified_base
<222> (14)..(14)
<223> 3' Guanine as 2'-O-methyl

<400> 149
tcctggcggg gaagt 15

<210> 150
<211> 15
<212> DNA
<213> Artificial Sequence

<220>
<223> Synthetic Oligonucleotide

<220>
<221> modified_base
<222> (8)..(8)
<223> 7-Deaza-2'-O-methyl-G

<220>
<221> modified_base
<222> (12)..(12)
<223> 3' Adenine as 2'-O-methyl

<220>
<221> modified_base
<222> (13)..(13)
<223> 3' Adenine as 2'-O-methyl

<220>
<221> modified_base
<222> (14)..(14)
<223> 3' Guanine as 2'-O-methyl

<400> 150
tcctggcggg gaagt 15

<210> 151
<211> 15
<212> DNA
<213> Artificial Sequence

<220>

<223> Synthetic Oligonucleotide

<220>
<221> modified_base
<222> (8)..(8)
<223> I

<220>
<221> modified_base
<222> (12)..(12)
<223> 3′ Adenine as 2′-O-methyl

<220>
<221> modified_base
<222> (13)..(13)
<223> 3′ Adenine as 2′-O-methyl

<220>
<221> modified_base
<222> (14)..(14)
<223> 3′ Guanine as 2′-O-methyl

<400> 151
tcctggcggg gaagt 15

<210> 152
<211> 15
<212> DNA
<213> Artificial Sequence

<220>
<223> Synthetic Oligonucleotide

<220>
<221> modified_base
<222> (8)..(8)
<223> 7-Deazainosine

<220>
<221> modified_base
<222> (12)..(12)
<223> 3′ Adenine as 2′-O-methyl

<220>
<221> modified_base
<222> (13)..(13)
<223> 3′ Adenine as 2′-O-methyl

<220>
<221> modified_base
<222> (14)..(14)
<223> 3′ Guanine as 2′-O-methyl

<400> 152
tcctggcggg gaagt 15

<210> 153
<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deaza-dG

<400> 153
cctggcgggg aagt 14

<210> 154
<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deaza-2'-O-methyl-G

<400> 154
cctggcgggg aagt 14

<210> 155
<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> I

<400> 155
cctggcgggg aagt 14

<210> 156

<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deazainosine

<400> 156
cctggcgggg aagt 14

<210> 157
<211> 16
<212> DNA
<213> Artificial Sequence

<220>
<223> Synthetic Oligonucleotide

<220>
<221> modified_base
<222> (9)..(9)
<223> 7-Deaza-dG

<400> 157
ctcctggcgg ggaagt 16

<210> 158
<211> 16
<212> DNA
<213> Artificial Sequence

<220>
<223> Synthetic Oligonucleotide

<220>
<221> modified_base
<222> (9)..(9)
<223> 7-Deaza-2'-O-methyl-G

<400> 158
ctcctggcgg ggaagt 16

<210> 159
<211> 16
<212> DNA
<213> Artificial Sequence

<220>
<223> Synthetic Oligonucleotide

<220>
<221> modified_base
<222> (9)..(9)
<223> I

<400> 159
ctcctggcgg ggaagt 16

<210> 160
<211> 16
<212> DNA
<213> Artificial Sequence

<220>
<223> Synthetic Oligonucleotide

<220>
<221> modified_base
<222> (9)..(9)
<223> 7-Deazainosine

<400> 160
ctcctggcgg ggaagt 16

<210> 161
<211> 18
<212> DNA
<213> Artificial Sequence

<220>
<223> Synthetic Oligonucleotide

<220>
<221> modified_base
<222> (11)..(11)
<223> 7-Deaza-dG

<400> 161
tgctcctggc ggggaagt 18

<210> 162
<211> 18
<212> DNA
<213> Artificial Sequence

<220>
<223> Synthetic Oligonucleotide

<220>
<221> modified_base
<222> (11)..(11)
<223> 7-Deaza-2’-O-methyl-G

<400> 162
tgctcctggc ggggaagt 18

<210> 163
<211> 18
<212> DNA
<213> Artificial Sequence

<220>
<223> Synthetic Oligonucleotide

<220>
<221> modified_base
<222> (11)..(11)
<223> I

<400> 163
tgctcctggc ggggaagt 18

<210> 164
<211> 18
<212> DNA
<213> Artificial Sequence

<220>
<223> Synthetic Oligonucleotide

<220>
<221> modified_base
<222> (11)..(11)
<223> 7-Deazainosine

<400> 164
tgctcctggc ggggaagt 18

<210> 165
<211> 18
<212> DNA
<213> Artificial Sequence

<220>
<223> Synthetic Oligonucleotide

<220>
<221> modified_base
<222> (11)..(11)
<223> 7-Deaza-dG

<400> 165
ttttcctggc ggggaagt 18

<210> 166
<211> 18
<212> DNA
<213> Artificial Sequence

<220>
<223> Synthetic Oligonucleotide

<220>
<221> modified_base

<222> (11)..(11)
<223> 7-Deaza-2'-O-methyl-G

<400> 166
ttttcctggc ggggaagt 18

<210> 167
<211> 18
<212> DNA
<213> Artificial Sequence

<220>
<223> Synthetic Oligonucleotide

<220>
<221> modified_base
<222> (11)..(11)
<223> I

<400> 167
ttttcctggc ggggaagt 18

<210> 168
<211> 18
<212> DNA
<213> Artificial Sequence

<220>
<223> Synthetic Oligonucleotide

<220>
<221> modified_base
<222> (11)..(11)
<223> 7-Deazainosine

<400> 168
ttttcctggc ggggaagt 18

<210> 169
<211> 19
<212> DNA
<213> Artificial Sequence

<220>
<223> Synthetic Oligonucleotide

<220>
<221> modified_base
<222> (9)..(9)
<223> 7-Deaza-dG

<400> 169
ctcctggcgg ggaagtttt 19

<210> 170
<211> 19
<212> DNA
<213> Artificial Sequence

<220>
<223> Synthetic Oligonucleotide

<220>
<221> modified_base
<222> (9)..(9)
<223> 7-Deaza-2'-O-methyl-G

<400> 170
ctcctggcgg ggaagtttt 19

<210> 171
<211> 19
<212> DNA
<213> Artificial Sequence

<220>
<223> Synthetic Oligonucleotide

<220>
<221> modified_base
<222> (9)..(9)
<223> I

<400> 171
ctcctggcgg ggaagtttt 19

<210> 172
<211> 19
<212> DNA
<213> Artificial Sequence

<220>
<223> Synthetic Oligonucleotide

<220>
<221> modified_base
<222> (9)..(9)
<223> 7-Deazainosine

<400> 172
ctcctggcgg ggaagtttt 19

<210> 173
<211> 14
<212> DNA
<213> Artificial Sequence

<220>
<223> Synthetic Oligonucleotide

<400> 173
ctcccgcgcg cggg 14

<210> 174
<211> 14
<212> DNA

<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deaza-dG

<400> 174
cctggcgggg aagt 14

<210> 175
<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (7)..(7)
<223> 7-Deaza-dG

<220>
<221> modified_base
<222> (11)..(11)
<223> 5-Iododeoxyuridine

<400> 175
cctggcgggg uagt 14

<210> 176
<211> 14
<212> DNA
<213> Artificial Sequence

<220>
<223> Synthetic Oligonucleotide

<220>
<221> modified_base
<222> (6)..(6)
<223> 5-Methyl-dC

<220>
<221> modified_base
<222> (11)..(11)

<223>  5-Iododeoxyuridine

<400>  176
cctggcgggg uagt                                                                  14

<210>  177
<211>  14
<212>  DNA
<213>  Artificial Sequence

<220>
<223>  Synthetic Oligonucleotide

<220>
<221>  modified_base
<222>  (7)..(7)
<223>  7-Deaza-dG

<220>
<221>  modified_base
<222>  (11)..(11)
<223>  5-Iododeoxyuridine

<400>  177
cctggcgggg uagt                                                                  14

<210>  178
<211>  14
<212>  DNA
<213>  Artificial Sequence

<220>
<223>  Synthetic Oligonucleotide

<220>
<221>  modified_base
<222>  (11)..(11)
<223>  5-Iododeoxyuridine

<400>  178
cctggcgggg uagt                                                                  14

<210>  179
<211>  14
<212>  DNA
<213>  Artificial Sequence

<220>
<223>  Synthetic Oligonucleotide

<220>
<221>  modified_base
<222>  (6)..(6)
<223>  5-Propinyl-C

<220>
<221>  modified_base
<222>  (7)..(7)
<223>  7-Deaza-dG

<400> 179
cctggcgggg aagt 14

<210> 180
<211> 14
<212> DNA
<213> Artificial Sequence

<220>
<223> Synthetic Oligonucleotide

<220>
<221> modified_base
<222> (6)..(6)
<223> 5-Propinyl-C

<220>
<221> modified_base
<222> (7)..(7)
<223> 7-Deaza-dG

<220>
<221> modified_base
<222> (11)..(11)
<223> 5-Iododeoxyuridine

<400> 180
cctggcgggg uagt 14

<210> 181
<211> 14
<212> DNA
<213> Artificial Sequence

<220>
<223> Synthetic Oligonucleotide

<220>
<221> modified_base
<222> (6)..(6)
<223> 5-Propinyl-C

<220>
<221> modified_base
<222> (11)..(11)
<223> 5-Iododeoxyuridine

<400> 181
cctggcgggg uagt 14

<210> 182
<211> 14
<212> DNA
<213> Artificial Sequence

<220>
<223> Synthetic Oligonucleotide

<220>
<221> misc_feature
<222> (6)..(6)
<223> 5-Octadienyl

<220>
<221> modified_base
<222> (7)..(7)
<223> 7-Deaza-dG

<400> 182
cctggngggg aagt 14

<210> 183
<211> 14
<212> DNA
<213> Artificial Sequence

<220>
<223> Synthetic Oligonucleotide

<220>
<221> misc_feature
<222> (6)..(6)
<223> 5-Octadienyl

<220>
<221> modified_base
<222> (7)..(7)
<223> 7-Deaza-dG

<220>
<221> modified_base
<222> (11)..(11)
<223> 5-Iododeoxyuridine

<400> 183
cctggngggg uagt 14

<210> 184
<211> 14
<212> DNA
<213> Artificial Sequence

<220>
<223> Synthetic Oligonucleotide

<220>
<221> misc_feature
<222> (6)..(6)
<223> 5-Octadienyl

<220>
<221> modified_base
<222> (11)..(11)
<223> 5-Iododeoxyuridine

<400> 184
cctggngggg uagt 14

<210>   185
<211>   14
<212>   DNA
<213>   Artificial Sequence

<220>
<223>   Synthetic Oligonucleotide

<220>
<221>   modified_base
<222>   (6)..(6)
<223>   5-Bromo-dC

<220>
<221>   modified_base
<222>   (7)..(7)
<223>   7-Deaza-dG

<400>   185
cctggcgggg aagt   14

<210>   186
<211>   14
<212>   DNA
<213>   Artificial Sequence

<220>
<223>   Synthetic Oligonucleotide

<220>
<221>   modified_base
<222>   (6)..(6)
<223>   5-Bromo-dC

<220>
<221>   modified_base
<222>   (7)..(7)
<223>   7-Deaza-dG

<220>
<221>   modified_base
<222>   (11)..(11)
<223>   5-Iododeoxyuridine

<400>   186
cctggcgggg uagt   14

<210>   187
<211>   14
<212>   DNA
<213>   Artificial Sequence

<220>
<223>   Synthetic Oligonucleotide

<220>
<221>   modified_base
<222>   (6)..(6)
<223>   5-Bromo-dC

<220>
<221> modified_base
<222> (11)..(11)
<223> 5-Iododeoxyuridine

<400> 187
cctggcgggg uagt 14

<210> 188
<211> 14
<212> DNA
<213> Artificial Sequence

<220>
<223> Synthetic Oligonucleotide

<220>
<221> modified_base
<222> (6)..(6)
<223> 5-Iodo-dC

<220>
<221> modified_base
<222> (7)..(7)
<223> 7-Deaza-dG

<400> 188
cctggcgggg aagt 14

<210> 189
<211> 14
<212> DNA
<213> Artificial Sequence

<220>
<223> Synthetic Oligonucleotide

<220>
<221> modified_base
<222> (6)..(6)
<223> 5-Iodo-dC

<220>
<221> modified_base
<222> (7)..(7)
<223> 7-Deaza-dG

<220>
<221> modified_base
<222> (11)..(11)
<223> 5-Iododeoxyuridine

<400> 189
cctggcgggg uagt 14

<210> 190
<211> 14
<212> DNA
<213> Artificial Sequence

<220>
<223> Synthetic Oligonucleotide

<220>
<221> modified_base
<222> (6)..(6)
<223> 5-Iodo-dC

<220>
<221> modified_base
<222> (11)..(11)
<223> 5-Iododeoxyuridine

<400> 190
cctggcgggg uagt 14

<210> 191
<211> 14
<212> DNA
<213> Artificial Sequence

<220>
<223> Synthetic Oligonucleotide

<220>
<221> modified_base
<222> (6)..(6)
<223> LNA-5-methyl-C

<220>
<221> modified_base
<222> (7)..(7)
<223> 7-Deaza-dG

<400> 191
cctggcgggg aagt 14

<210> 192
<211> 14
<212> DNA
<213> Artificial Sequence

<220>
<223> Synthetic Oligonucleotide

<220>
<221> modified_base
<222> (6)..(6)
<223> LNA-5-methyl-C

<220>
<221> modified_base
<222> (7)..(7)
<223> 7-Deaza-dG

<220>
<221> modified_base
<222> (11)..(11)
<223> 5-Iododeoxyuridine

<400> 192
cctggcgggg uagt 14

<210> 193
<211> 14
<212> DNA
<213> Artificial Sequence

<220>
<223> Synthetic Oligonucleotide

<220>
<221> modified_base
<222> (6)..(6)
<223> LNA-5-methyl-C

<220>
<221> modified_base
<222> (11)..(11)
<223> 5-Iododeoxyuridine

<400> 193
cctggcgggg uagt 14

<210> 194
<211> 14
<212> DNA
<213> Artificial Sequence

<220>
<223> Synthetic Oligonucleotide

<220>
<221> modified_base
<222> (6)..(6)
<223> 5-Methyl-dC

<400> 194
cctggcgggg aagt 14

<210> 195
<211> 14
<212> DNA
<213> Artificial Sequence

<220>
<223> Synthetic Oligonucleotide

<220>
<221> modified_base
<222> (6)..(6)
<223> 5-Methyl-dC

<400> 195
cctggcgtgg aagt 14

<210> 196
<211> 14

<212> DNA
<213> Artificial Sequence

<220>
<223> Synthetic Oligonucleotide

<220>
<221> modified_base
<222> (6)..(6)
<223> 5-Methyl-dC

<400> 196
cttggcgggg aagt 14

<210> 197
<211> 14
<212> DNA
<213> Artificial Sequence

<220>
<223> Synthetic Oligonucleotide

<400> 197
cctggcgggg aagt 14

<210> 198
<211> 14
<212> DNA
<213> Artificial Sequence

<220>
<223> Synthetic Oligonucleotide

<220>
<221> modified_base
<222> (7)..(7)
<223> 7-Deaza-dG

<400> 198
cctggcgggg aagt 14

<210> 199
<211> 18
<212> DNA
<213> Artificial Sequence

<220>
<223> Synthetic Oligonucleotide

<220>
<221> modified_base
<222> (11)..(11)
<223> 7-Deaza-dG

<400> 199
tgctcctgga ggggttgt 18

## Description

### I. Field of the Invention

The invention generally relates to the field of immunology and immunotherapy, and more specifically to immune regulatory oligonucleotide (IRO) compositions and their use for inhibition and/or suppression of Toll-like Receptor-mediated immune responses.

### II. Background of the invention

Toll-like receptors (TLRs) are present on certain cells of the immune system and have been shown to be involved in the innate immune response. In vertebrates, this family consists of proteins called TLR1 to TLR10, which are known to recognize pathogen associated molecular patterns from bacteria, fungi, parasites, and viruses. TLRs are a key means by which mammals recognize and mount an immune response to foreign molecules and also provide a means by which the innate and adaptive immune responses are linked. TLRs have also been shown to play a role in the pathogenesis of many diseases, including autoimmunity, infectious disease, and inflammation and the regulation of TLR-mediated activation. By using appropriate agents this may provide a means for disease intervention.

Some TLRs are located on the cell surface to detect and initiate a response to extracellular pathogens. Other TLRs are located inside the cell to detect and initiate a response to intracellular pathogens.

Certain unmethylated CpG motifs present in bacterial and synthetic DNA have been shown to activate the immune system and induce antitumor activity. Other studies using antisense oligonucleotides containing unmethylated CpG dinucleotides have been shown to stimulate immune responses. Subsequent studies demonstrated that TLR9 recognizes unmethylated CpG motifs present in bacterial and synthetic DNA. Other modifications of CpG-containing phosphorothioate oligonucleotides can also affect their ability to act as modulators of immune response through TLR9. In addition, structure activity relationship studies have allowed identification of synthetic motifs and novel DNA-based compounds that induce specific immune response profiles that are distinct from those resulting from unmethylated CpG dinucleotides.

The selective localization of TLRs and the signaling generated therefrom, provides some insight into their role in the immune response. The immune response involves both an innate and an adaptive response based upon the subset of cells involved in the response. For example, the T helper (Th) cells involved in classical cell-mediated functions such as delayed- type hypersensitivity and activation of cytotoxic T lymphocytes (CTLs) are Th1 cells. This response is the body's innate response to pathogens or their antigens, respectively (e.g. viral infections, intracellular pathogens, and tumor cells), and results in a secretion of IFN- gamma and a concomitant activation of CTLs. Alternatively, the Th cells involved as helper cells for B-cell activation are Th2 cells. Th2 cells have been shown to be activated in response to bacteria and parasites and may mediate the body's adaptive immune response (e.g. IgE production and eosinophil activation) through the secretion of IL-4, IL-5 and IL-10. The type of immune response is influenced by the cytokines produced in response to antigen exposure and the differences in the cytokines secreted by Th1 and Th2 cells may be the result of the different biological functions of these two subsets. In certain diseases, such as asthma and allergies, the bodies Th1/Th2 balance is shifted towards a Th2 environment.

While activation of TLRs is involved in mounting an immune response, an uncontrolled stimulation of the immune system through TLRs may exacerbate certain diseases in immune compromised subjects. Several groups have already shown the use of synthetic oligodeoxyoligonucleotides (ODNs) as inhibitors of inflammatory cytokines. These inhibitory ODN require two triplet sequences, a proximal "CCT" triplet and a distal "GGG" triplet. In addition to these triplet-containing inhibitory ODNs, several groups have reported other specific DNA sequences that could inhibit TLR-9-mediated activation by CpG-containing ODNs. These "inhibitory" or "suppressive" motifs are rich in poly "G" (e.g. "GGGG") or "GC" sequences, tend to be methylated, and are present in the DNA of mammals and certain viruses.

Other studies have called into question the view that poly G containing ODNs are acting as antagonists of TLRs. It has been demonstrated that administering CpG oligonucleotides containing GGGG strings have potent antiviral and anticancer activity, and further that administration of these compounds will cause an increase in serum IL-12 concentration. Further, CpG oligos containing polyG sequences are known to induce immune responses through TLR9 activation.

In addition, oligonucleotides containing guanosine strings have been shown to form tetraplex structures (tetrads), act as aptamers and inhibit thrombin activity. Thus it is not clear whether single-stranded or multiple-stranded structures, later forming inhomogeneous high molecular aggregates, are effective at suppressing TLR9 activation. However, the presence of G-tetrads makes their immunological and pharmacocological behavior unpredictable. The presence of polyG sequences in an oligonucleotide may also change its intracellular concentration and localization.

Reaction to certain motifs in bacterial DNA is an important function of natural immunity of vertebrates. Bacterial DNA has long been known to be mitogenic for mammalian B lymphocytes (B cells), whereas mammalian DNA generally is not. The discovery that this immune recognition was directed to specific DNA sequences centered on a motif containing an unmethylated CpG dinucleotide opened the field to molecular immunologic approaches.

CpG sites or CG sites are regions of DNA where a cytosine nucleotide occurs next to a guanine nucleotide in the linear sequence of bases along its length. "CpG" is shorthand for "-C-phosphate-G-", that is, cytosine and guanine separated by only one phosphate; phosphate links any two nucleosides together in DNA. The "CpG" notationis used to distinguish this linear sequence from the CG base-pairing of cytosine and guanine.

Cytosines in CpG dinucleotides can be methylated to form e.g. 5-methylcytosine. In mammals, methylating the cytosine within a gene can turn the gene off. In mammals, 70% to 80% of CpG cytosines are methylated.

The immunostimulatory effects of so-called CpG DNA can be reproduced using synthetic oligodeoxynucleotides (ODN) containing CpG dinucleotides in the context of certain preferred flanking sequence, a CpG motif. The optimal sequence context has been found to be the hexanucleotides GTCpGTT for human TLR9 and GACpGTT for murine TLR9, respectively. CpG-containing ODN (CpG-ODN) have been reported to exert a number of effects on various types of cells of the immune system, including protecting primary B cells from apoptosis, promotion of cell cycle entry, and skewing an immune response toward a Th1-type immune response, e.g., induction of interleukin 6 (IL-6), interleukin 12 (IL-12), gamma interferon (IFN-$\gamma$), activation of antigen-specific cytolytic T lymphocytes (CTL), and induction in the mouse of IgG2a.

It has been reported that the immuno- modulatory effects of CpG DNA involve signaling by Toll-like receptor 9 (TLR9). It is believed that CpG DNA is internalized into a cell via a sequence-nonspecific pathway and traffics to the endosomal compartment, where it interacts with TLR9 in a sequence-specific manner. TLR9 signaling pathways lead to induction of a number of immune-function related genes, including notably NF-KB-mediated induction of cytokine and chemokine secretion, among others.

The TLRs are a large family of receptors that recognize specific molecular structures that are present in pathogens (pathogen-associated molecular patterns or PAMPs) and are also termed pattern recognition receptors (PRRs). Immune cells expressing PRRs are activated upon recognition of PAMPs and trigger the generation of optimal adaptive immune responses. PRRs consisting of 10 different TLR subtypes, TLR1 to TLR10, have been described. Such TLRs have been described to be involved in the recognition of double-stranded RNA (TLR3), lipopolysaccharide (LPS) (TLR4), bacterial fiagellin (TLR5), small anti-viral compounds as well as single-stranded RNA (TLR7 and TLR8), and bacterial DNA or CpG ODN (TLR9). Reviewed in Uhlmann et al. (2003) Curr Opin Drug Discov Deve/ 6:204-17.

US 2005/0239733 describes oligonucleotides with immune inhibitory activity which comprise the sequence Xa**CC** $N_1N_2N_3Y_bN_4$**GGG**$Z_c$. These sequences contain the motif 5' CC-Li-GGG in which the linker (Li) has the meaning $N_1N_2N_3Y_bN_4$, wherein $N_1$, $N_2$, $N_3$ and $N_4$ are each independently any nucleotide and $Y_b$ may be any nucleotide sequence wherein b is an integer between 8 and 21.

WO 2011/005942 discloses oligonucleotide-based TLR antagonists containing a modified immune stimulatory motif, having the structure 5-Nm-$N_3N_2N_1$CG$N_1N_2N_3$-Nm-3', wherein CG is the modified immune stimulatory motif and C is cytosine, or a pyrimidine nucleotide derivative and G is guanosine or a purine nucleotide derivative.

WO 2011/005942 discloses an oligonucleotide motif which is immune stimulatory in a parent oligonucleotide, but not in a derivative oligonucleotide, wherein the derivative oligonucleotide is based upon the parent oligonucleotide, but has one or more modifications to the oligonucleotide motif that reduce or eliminate immune stimulation.

## III. Summary of the invention

It has been found that certain nucleic acid molecules selectively inhibit signaling mediated by Toll-like receptors TLR9, TLR8, and TLR7. These nucleic acid molecules are inhibitory oligodeoxynucleotides (I N H ODN) ranging in length from 2 to about 50, preferably 4 to 30, more preferred from 5 to 20 and especially preferred from 6 to 15 nucleotides. While certain of the inhibitory ODN are selectively inhibitory with respect to just one of TLR9, TLR8, TLR7 or TLR3, certain of the inhibitory ODN are selectively inhibitory with respect to two or more of TLR9, TLR8, TLR7 and TLR3. The inhibitory ODN can be used alone, in combination with one another, or in combination with another agent, e.g., a small molecule TLR inhibitor, an immunosuppressive molecule, such as glucocorticoids, cytostatics or antibodies, or even with an immunostimulatory CpG nucleic acid molecule or TLR agonist, to shape an immune response in vivo or in vitro.

There is a need for alternative inhibitory oligonucleotides which have a good and preferably superior biological activity.

The present invention discloses inhibitory oligonucleotides having the general formula:

$$X_1C\ C\ N_1N_2\ N_3\ X_2N_4N_5\ G\ G\ G\ N_6\ X_3N_7 \qquad (I)$$

wherein

C is cytidine or a derivative thereof, whereby the cytidine derivative is selected from the group consisting of 5-methylcytidine, a cytidine-like nucleotide having a chemical modification involving the cytosine base, cytidine nucleoside sugar, or both the cytosine base and the cytidine nucleoside sugar, 2'-O-methylcytidine, 5-substitued-cytidine, 5-bromocytidine, 5-hydroxycytidine, ribocytidine and β-D-arabinofuranoside-cytidine, and 2'-fluoro- β-D-arabinofuranoside-cytidine.

G is guanosine or a derivative thereof, whereby the guanosine derivative is selected from the group consisting of 7-deazaguanosine, 2'-deoxy-7-deazaguanosine, 2'-O-methyl-7-deazaguanosine, inosine, 2'-deoxyinosine, 7-deaza-2'-deoxyinosine, a guanosine-like nucleotide having a chemical modification involving the guanine base, the guanosine nucleoside sugar or both the guanine base and the guanosine nucleoside sugar,

$X_1$ and $X_3$ is any nucleotide sequence with 0 to 12 bases and each nucleotide is independent of any other, $X_2$ is any nucleotide sequence having 0 to 3 nucleotides,

$N_1$, $N_2$ and $N_3$ are each independently any nucleotide,

$N_4$ and $N_7$ is a pyrimidine or a modified pyrimidine,

$N_5$ is a purin or a modified purin,

$N_6$ is a modified pyrimidine, A or a modified purin, with the proviso that

at least two of the nucleotides $N_4$, $N_5$, $N_6$ or $N_7$ are modified purins and/or modified pyrimidines.

In a preferred embodiment the inhibitory oligonucleotide having the general formula (I) have the above meaning, wherein

$X_1$ and $X_3$ is any nucleotide sequence with 0 to 6 bases and each nucleotide is independent of any other, $X_2$ is 0 or 1 nucleotide,

$N_1$, $N_2$ and $N_3$ are each independently any nucleotide,

$N_4$ and $N_7$ is a pyrimidine or a modified pyrimidine,

$N_5$ is a purin or a modified purin,

$N_6$ is a modified pyrimidine, A or a modified purin,

wherein at least two of the nucleotides $N_4$, $N_5$, $N_6$ or $N_7$ are modified purins or modified pyrimidines, and whereby the oligonucleotide comprises not ore or less than 20 nucleotides.

In a particularly preferred embodiment the inhibitory oligonucleotide has the general formula (II):

$X_1$C C T G G py pu G G G px A G py (III)

wherein

C is cytidine or a derivative thereof as defined above,

G is guanosine or a derivative thereof as defined above,

$X_1$ is any nucleotide or no nucleotide,

py is a pyrimidine or a modified pyrimidine nucleotide,

pu is a purin or a modified purin nucleotide,

px is a modified pyrimidine, A or a modified purin,

wherein at least two of the nucleotides py, pu and px are modified purins or modified pyrimidines selected from the group consisting of 7-deaza-desoxyguanosine, 7-deaza-2'-O-methylguanosine, inosine, diaminopurin, 6-thio-desoxyguanosine, 6-O-methyl-desoxyguanosine, 7-deaza-inosine, 7-deaza-7-iododesoxyguanosine, 7-aminopropargyldesoxaguanosine, 2-fluoro-cytosine, 5-methylcytosine.

Particularly preferred are inhibitory oligonucleotides having formula (II), wherein

Py is 5-substituted cytidine, selected from the group consisting of 5-methyl-dC, 5-bromo-dC and 5-octadienyl-dC,

Pu is a 7-deaza purin derivative, selected from the group consisting of 7-deaza-dG, 7-deaza-2'-O-methyl-G, inosine and 7-deaza-inosine and Px is dA or 5-iodo-dU.

Px is dA, 5-substituted deoxyuridine, and 5-iodo-uridine.

In a particular preferred embodiment the inhibitory oligonucleotides having general formula (II) Py has the meaning of 5-methyl-dC and/or Pu has the meaning of 7-deaza-dG, 7-deaza-2'-O-methyl-G or 7-deaza-inosine and/or Px has the meaning 5-substituted-2'-deoxyuridine.

In another preferred embodiment the inhibitory oligonucleotides having general formula (II), X1 is zero.

In a particular preferred embodiment the inhibitory oligonucleotides having general formula (II), Px has the meaning 5-substituted-2'-deoxyuridine.

In a particular preferred embodiment the inhibitory oligonucleotides having general formula (II) Py has the meaning of 5-methyl-dC and/or Pu has the meaning of 7-deaza-dG, 7-deaza-2'-O-methyl-G or 7-deaza-inosine, Px has the meaning 5-substituted-2'-deoxyuridine, and X1 is zero.

In another preferred embodiment, the inhibitory oligonucleotide has one of the SEQ ID 25064, 25070, 25071, 25077, 25078, 25079, 25080, 106918, 106919, 106920, 106921, 106924, 106927, 106929, 106930, 106931, or 106937.

In an especially preferred embodiment, the inhibitory oligonucleotide has the SEQ ID 25078.

In an especially preferred embodiment, the inhibitory oligonucleotide has the SEQ ID 106918.

In an especially preferred embodiment, the inhibitory oligonucleotide has the SEQ ID 106919.

In another preferred embodiment, the inhibitory oligonucleotide has the SEQ ID 106930.

Especially preferred inhibitory oligonucleotides have the general formula (III):

$X_1$C C T G G py pu G G G (III)

wherein C, G, $N_1$, py, and pu have the meaning as defined above.

Particularly preferred are inhibitory oligonucleotides having formular (III), wherein

Py is 5-substituted cytidine, selected from the group consisting of 5-methyl-dC, 5-bromo-dC and 5-octadienyl-dC, Pu is a 7-deaza purin derivative, selected from the group consisting of 7-deaza-dG, 7-deaza-2'-O-methyl-G, inosine and 7-deaza-inosine and Px is dA or 5-iodo-dU.

In a particular preferred embodiment the inhibitory oligonucleotide having general formula (III) Py has the meaning of 5-methyl-dC and/or Pu has the meaning of 7-deaza-dG, 7-deaza-2'-O-methyl-G or 7-deaza-inosine and/or $X_1$ has the meaning: no nucleotide.

In another preferred embodiment, the inhibitory oligonucleotide has the general formula IV

$X_1$ A A T GG py pu G G G px A G py (V)

where

Py is 5-substituted cytidine, selected from the group consisting of 5-methyl-dC, 5-bromo-dC and 5-octadienyl-dC, Pu is a 7-deaza purin derivative, selected from the group consisting of 7-deaza-dG, 7-deaza-2'-O-methyl-G, inosine and 7-deaza-inosine and Px is dA or 5-iodo-dU.
Px is dA, 5-substituted deoxyuridine, and 5-iodo-uridine and $X_1$ is any nucleotide or no nucleotide.

In a particular preferred embodiment, the inhibitory oligonucleotide of the formula II is a TLR7/TLR9 antagonist.

In a particular preferred embodiment, the inhibitory oligonucleotide of the formula III is a TLR9 antagonist.

In a particular preferred embodiment, the inhibitory oligonucleotide of the formula IV is a TLR7 antagonist.

In a particular preferred embodiment, the inhibitory oligonucleotide of the formula II is a TLR7/TLR9/TLR3 antagonist.

In a particular preferred embodiment, the inhibitory oligonucleotide of the formula II is a TLR3 antagonist.

In a particular preferred embodiment, the inhibitory oligonucleotide of the formula II is a TLR7 antagonist.

In a particular preferred embodiment, the inhibitory oligonucleotide of the formula II is a TLR9 antagonist.

In a particular preferred embodiment, the inhibitory oligonucleotide of the formula II is a TLR3/TLR7/TLR8/TLR9 antagonist.

In a particular preferred embodiment, the inhibitory oligonucleotide of the formula II is a TLR7/TLR8/TLR9 antagonist.

The inhibitory oligonucleotides of the present invention having the formula as described in more detail above are preferably used as a TLR antagonist with strongly enhanced potency. The inhibitory oligonucleotides of the invention may be used to treat a number of conditions that involve an innate immune response or a Th1-like immune response, including autoimmune diseases, inflammation, allograft rejection, graft-versus host disease, cancer, infection and sepsis. The inhibitory oligonucleotides can be used in the prevention of autoimmune disorders, an airway inflammation, inflammatory disorders, infectious diseases, skin disorders(e.g. psoriasis), allergy, asthma or a disease caused by a pathogen such as a bacterium or a virus. Autoimmune diseases include e.g. systemic lupus erythematosus (SLE), inflammatory bowel disease, Crohn's disease, ulcerative colitis, rheumatoid arthritis, multiple sclerosis, and diabetes mellitus. TLR signaling has been linked to neurogenesis and was found to be involved in the pathogenesis of neurodegenerative diseases. Thus interfering with TLR signaling in glial cells may also be used to prevent or treat neurodegenerative diseases such as Alzheimer's disease, prion diseases, amyotrophic lateral sclerosis, and Parkinson's disease.

The inhibitor oligonucleotide can be used in a pharmaceutical composition. Such composition may contain only the oligonucleotide or alternatively physiologically acceptable additives and/or carriers which are required for a proper pharmaceutical administration such as fillers, expanders. The pharmaceutical composition can be in the form of tablets, capsules, dragees or in the form of solutions suitable for injection or infusion. Preferred routes of admistration are subcutaneous, intradermal, intraperitoneal or intrathecal injections. In case of lung disorders, but also other disorders, administration of the antagonists by inhalation may be preferred.

Pharmaceutical compositions can be used to prevent or treat autoimmune disorders, an airway inflammation, inflammatory disorders, infectious diseases, skin disorders, allergy, asthma or a disease caused by a pathogen such as a bacterium or a virus.

In the present disclosure of the invention terms and definitions having the following meanings are used if not stated otherwise:

The term *"oligonucleotide"* generally refers to a polynucleotide comprising a plurality of linked nucleoside units. Such oligonucleotides can be obtained from existing nucleic acid sources, including genomic or cDNA, but are preferably produced by synthetic methods. In preferred embodiments each nucleoside unit can encompass various chemical modifications and substitutions as compared to wild-type oligonucleotides, including but not limited to modified nucleoside base and/or modified sugar unit. Examples of chemical modifications are known to the person skilled in the art and are described, for example, in Uhlmann E et al. (1990) Chem. Rev. 90:543; "Protocols for Oligonucleotides and Analogs". Nucleotides, their derivatives and the synthesis therof is described in Habermehl et al., Naturstoffchemie, 3rd edition, Springer, 2008.

The nucleoside residues can be coupled to each other by any of the numerous known internucleoside linkages. Such internucleoside linkages include, without limitation, phosphodiester, phosphorothioate, phosphorodithioate, alkylphosphonate, alkylphosphonothioate, phosphotriester, phosphoramidate, phosphonoacetate, phosphonoacetate esters, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide" also encompasses polynucleosides having one or more stereospecific internucleoside linkage (e.g., (Rp)- or (Sp)-phosphorothioate, alkylphosphonate, or phosphotriester linkages). As used herein, the terms "oligonucleotide" and "dinucleotide" are expressly intended to include polynucleosides and dinucleosides having any such internucleoside linkage, whether or not the linkage comprises a phosphate group. In certain preferred embodiments, these internucleoside linkages may be phosphodiester, phosphorothioate, or phosphorodithioate linkages, or combinations thereof.

The term "2'-substituted ribonucleoside" or "2'-substituted arabinoside" generally includes ribonucleosides or arabinonucleosides in which the hydroxyl group at the 2' position of the pentose moiety is substituted to produce a 2'-substituted or 2'-substituted ribonucleoside. In certain embodiments, such substitution comprises ribonucleosides substituted with a lower hydrocarbyl group containing 1-6 saturated or unsaturated carbon atoms, or with a halogen atom, or with an aryl group having 6-10 carbon atoms, wherein such hydrocarbyl, or aryl group may be unsubstituted or may be substituted, e.g., with halo, hydroxy, trifluoromethyl, cyano, nitro, acyl, acyloxy, alkoxy, carboxyl, carboalkoxy, or amino groups. Examples of 2'-0-substituted ribonucleosides or 2'-0-substituted-arabinosides include, without limitation 2'-amino, 2'-fluoro, 2'-allyl, 2'-0-alkyl and 2'-propargyl ribonucleosides or arabinosides, 2'-fluroarabino nucleosides (FANA), 2'-0-methylribonucleosides or 2'-0-methylarabinosides and 2'-0-methoxyethoxyribonucleosides or 2'-0-methoxyethoxyarabinosides.

The term " 3' ", when used directionally, generally refers to a region or position in a polynucleotide or oligonucleotide 3' (downstream) from another region or position in the same polynucleotide or oligonucleotide.

The term " 5' ", when used directionally, generally refers to a region or position in a polynucleotide or oligonucleotide 5' (upstream) from another region or position in the same polynucleotide or oligonucleotide.

The term "about" generally means that the exact number is not critical. Thus, the number of nucleoside residues in the oligonucleotides is not critical, and oligonucleotides having one or two fewer nucleoside residues, or from one to several additional nucleoside residues are contemplated as equivalents of each of the embodiments described above.

The term "agonist" generally refers to a substance that binds to a receptor of a cell and induces a response. An agonist often mimics the action of a naturally occurring substance such as a ligand.

The term "antagonist" generally refers to a substance that attenuates the effects of an agonist.

The term "adjuvant" generally refers to a substance which, when added to an immunogenic agent such as vaccine or antigen, enhances or potentiates an immune response to the agent in the recipient host upon exposure to the mixture.

The term "airway inflammation" generally includes, without limitation, asthma.

The term "allergen" generally refers to an antigen or antigenic portion of a molecule, usually a protein, which elicits an allergic response upon exposure to a subject. Typically the subject is allergic to the allergen as indicated, for instance, by a suitable test or any method known in the art. A molecule is said to be an allergen even if only a small subset of subjects exhibit an allergic immune response upon exposure to the molecule.

The term "allergy" generally refers to an inappropriate immune response characterized by inflammation and includes, without limitation, food allergies and respiratory allergies.

The term "antigen" generally refers to a substance that is recognized and selectively bound by an antibody or by a T cell antigen receptor, resulting in induction of an immune response. Antigens may include but are not limited to peptides, proteins, nucleosides, nucleotides, and combinations thereof. Antigens may be natural or synthetic and generally induce an immune response that is specific for that antigen.

The term "autoimmune disorder" generally refers to disorders in which "self" components undergo attack by the immune system.

The term "TLR-mediated disease" or TLR-mediated disorder" generally means any pathological condition for which activation of one or more TLRs is a contributing factor. Such conditions include but are not limited, autoimmune disorders (e.g. psoriasis), an airway inflammation, inflammatory disorders, infectious diseases, skin disorders, allergy, asthma or a disease caused by a pathogen such as a bacterium or a virus.

The term "physiologically acceptable" generally refers to a material that does not interfere with the effectiveness of an IRO compound and that is compatible with a biological system such as a cell, cell culture, tissue, or organism. Preferably, the biological system is a living organism, such as a vertebrate.

The term "carrier" generally encompasses any excipient, diluent, filler, salt, buffer, stabilizer, solubilizer, oil, lipid, lipid containing vesicle, microspheres, liposomal encapsulation, or other material well known in the art for use in pharmaceutical formulations. It will be understood that the characteristics of the carrier, excipient, or diluent will depend on the route of administration for a particular application. The preparation of pharmaceutically acceptable formulations containing these materials is described in, e.g., Remington's Pharmaceutical Sciences, 18th Edition, ed. A. Gennaro, Mack Publishing Co., Easton, Pa., 1990.

The term "co-administration" generally refers to the administration of at least two different substances sufficiently close in time to modulate an immune response. Co-administration refers to simultaneous administration, as well as temporally spaced order of up to several days apart, of at least two different substances in any order, either in a single dose or separate doses.

The term "complementary" generally means having the ability to hybridize to a nucleic acid. Such hybridization is ordinarily the result of hydrogen bonding between complementary strands, preferably to form Watson-Crick or Hoogsteen base pairs, although other modes of hydrogen bonding, as well as base stacking can also lead to hybridization.

The term an "effective amount" or a "sufficient amount" generally refers to an amount sufficient to affect a desired biological effect, such as beneficial results. Thus, an "effective amount" or "sufficient amount" will depend upon the context in which it is being administered. In the context of administering a composition that modulates an immune response to a co-administered antigen, an effective amount of an IRO compound and antigen is an amount sufficient to achieve the desired modulation as compared to the immune response obtained when the antigen is administered alone. An effective amount may be administered in one or more administrations.

The term "in combination with" generally means in the course of treating a disease or disorder in a patient, administering an IRO compound and an agent useful for treating the disease or disorder that does not diminish the immune modulatory effect of the IRO compound. Such combination treatment may also include more than a single administration of an IRO compound and/or independently an agent. The administration of the IRO compound and/or the agent may be by the same or different routes.

The term "individual" or "subject" or "vertebrate" generally refers to a mammal, such as a human. Mammals generally include, but are not limited to, humans.

The term "nucleoside" generally refers to compounds consisting of a sugar, usually ribose or deoxyribose, and a purine or pyrimidine base.

The term "nucleotide" generally refers to a nucleoside comprising a phosphate group attached to the sugar.

As used herein, the term "pyrimidine nucleoside" or "py" refers to a nucleoside wherein the base component of the nucleoside is a pyrimidine base (e.g., cytosine (C) or thymine (T) or Uracil (U)). Similarly, the term "purine nucleoside" or "pu" refers to a nucleoside wherein the base component of the nucleoside is a purine base (e.g., adenine (A) or guanine (G)).

The terms "analog" or "derivative" can be used interchangeable to generally refer to any purine and/or pyrimidine nucleotide or nucleoside that has a modified base and/or sugar. A modified base is a base that is not guanine, cytosine, adenine, thymine or uracil. A modified sugar is any sugar that is not ribose or 2'deoxyribose and can be used in the backbone for an oligonucleotide.

The term "inhibiting" or "suppressing" generally refers to a decrease in a response or qualitative difference in a response, which could otherwise arise from eliciting and/or stimulation of a response.

The term "non-nucleotide linker"generally refers to any linkage or moiety that can link or be linked to the oligonucleotides other than through a nucleotide-containing linkage. Preferably such linker is from about 2 angstroms to about 200 angstroms in length.

The term "nucleotide linkage" generally refers to a direct 3'-5' linkage that directly connects the 3' and 5' hydroxyl groups of two nucleosides through a phosphorous-containing linkage.

The term "oligonucleotide motif' means an oligonucleotide sequence, including a dinucleotide. An "oligonucleotide motif that would be immune stimulatory, but for one or more modifications" means an oligonucleotide motif which is immune stimulatory in a parent oligonucleotide, but not in a derivative oligonucleotide, wherein the derivative oligonucleotide is based upon the parent oligonucleotide, but has one or more modifications.

The term CpG refers to a dinucleotide motif which in a certain sequence context (e.g. GT-CpG-TT or GA-CpG-TT) may be immune stimulatory and comprises cytosine or a cytosine analog and a guanine or a guanine analog.

In the present application and the sequence protocol abbreviations were used which have the following meaning:

| | |
|---|---|
| BC | 5-Bromo-dC |
| E | 7-Deaza-dG (identica to dE) |
| dE | 7-Deaza-dG |
| D | dspacer, abasic |
| DI | 7-Deaza-2'-deoxyinosine |
| dZ | 5-Methyl-dC |
| faC | 2'-FANA-C (FANA means 2'-fluoroarabino nucleic acid) |
| frA | 2'-Fluoro-A |
| frC | 2'-Fluoro-C |
| frG | 2'-Fluoro-G |
| I | 2'-Deoxy-inosine |
| J | C3 spacer |
| JC | 5-lodo-dC |
| JG | 7-Deaza-7-iodo-dG |
| JU | 5-lododeoxyuridine |
| mA | 2'-O-methyl-A |
| mC | 2'-O-methyl-C |
| mE | 7-Deaza-2'-O-methyl-G |
| mG | 2'-O-methyl-G |
| O | 8-Oxo-dG |
| ODC | 5-Octadienyl-dC |
| PC | 5-Propinyl-dC |
| PG | 7-Aminopropargyl-dG |
| Q | 8-Oxo-dA |
| V | 2,6-Diaminopurine nucleoside |
| ßA | LNA-A |
| ßG | LNA-G |
| ßZ | LNA-5-methyl-C |
| 6MG | 6-O-methyl-dG |
| 6TG | 6-Thio-dG |
| 8G | 7-Deaza-8-aza-dG |

The term "treatment" generally refers to an approach intended to obtain a beneficial or desired results, which may include alleviation of symptoms, or delaying or ameliorating a disease progression.

In a first aspect, the invention provides an immune inhibitory oligonucleotide (INH ODN) compound. The term "INH ODN" refers to an immune regulatory oligonucleotide compound that is an antagonist for one or more TLR, wherein the compound comprises an oligonucleotide motif CC-Li-GGG and at least two modifications, wherein the oligonucleotide motif would not be immune stimulatory (e.g., even if it contains an unmethylated CpG), provided that compound contains less than 4 consecutive unmodified guanosine nucleotides. Such modifications may be in the oligonucleotide 5' terminus, in a sequence flanking the oligonucleotide motif, and/or within the oligo- nucleotide motif. These modifications result in an IRO compound that suppresses TLR-modulated immune stimulation. Such modifications can be to the bases, sugar residues and/or the phosphate backbone of the nucleotides/nucleosides flanking the oligonucleotide motif or within the oligonucleotide motif.

Although the present invention encompasses oligonucleotide sequences which have modifications of the CG dinucleotide, such sequences do not have an immune stimulatory effect on TLR9. Contrary to the compounds disclosed in WO 2011/005942 the molecules of the present invention suppress TRL9 modulated immune stimulation. The compounds disclosed herein are immune inhibitory oligonucleotides (INH ODN).

In preferred embodiments the INH ODN compound is not an antisense oligonucleotide. Important aspects of the present invention are shown in the examples and the figures.

**Examples**

In three rounds of screening, novel TLR antagonists with modified nucleotide analogs have been identified which show strongly enhanced inhibitory activity as compared to the prototype TLR antagonist described in US 2005/0239733 as SEQ ID NO:4 having the sequence 5' dT*dC*dC*dT*dG*dG*dC*dG*dG*dG*dG*dA*dA*dG*dT.

The biological activity of the oligonucleotides disclosed in the present application were tested in an in vitro test which has been performed as follows:

Stably transfected HEK293 cells expressing human TLR9 or murine TLR9 or human TLR7 together with a 6xNF-kB-Luciferase reporter gene construct and the use thereof were extensively described in the literature (Bauer et al. (2001) PNAS 98(16), 9237-42). Heil et al. (2004) Science 303(5663), 1529-9, Jurk et al. (2006) Eur J. Immunol. 36(7), 1815-26).

Stable transfectants ($2.5x10^4$ cells/well) were plated overnight and incubated first with increasing amounts of inhibitory ODN followed by addition of the respective agonist. For human TLR9 0.5 μM ODN 10103 (sequence published in Luganini et al. (2008) Antimicrob. Agents Chemother. 52, 1111-1120.); for murine TLR9 0.5 μM ODN 1826 (as described in Bauer et al. (2001) PNAS 98(16), 9237-42) and for human TLR7 with 2 μM R848 (as published in Jurk et al (2006) Eur. J. Immunol 36(7), 1815-26) for 16h at 37°C in a humidified incubator (5% $CO_2$), Each data point was done in duplicate. Cells were lysed using OneGlo™, Promega, Madison, WI, USA and analysed after 10 min incubation at RT for luciferase gene activity.

Stimulation indices were calculated in reference to reporter gene activity of medium without addition of ODN. Activity of the TLR agonist alone was set to 100% and inhibition of activity in the presence of inhibitory ODN was calculated accordingly.

Example 1

Maximum increase in inhibitory activity was obtained in ODN with two to three chemical modifications. In the first round of screening, dG was replaced by 7-deaza-dG (*dE*), 7-deaza-2'-O-methyl-G (mE*), Inosin (I*), Diaminopurin (V*), 8-oxo-dG (O*) and various other nucleotide analogs. The ODN's synthesized and tested are shown in Fig. 1A - Fig.1E.

The ODN's contain mainly a single substitution between dC*dC* and dG*dG*dG*dG* whereby the substitution can also be located within dG*dG*dG*dG*.

The biological activity which has been measured as described above of the ODN's having one mutation (one dG* replaced by dE* in the *dG*dG*dG*dG* at various positions) is shown in Figure 2 and Figure 3). As can be seen from Figure 3 the best biological activity is obtained when the first G of the GGG motif is replaced by a modified nucleotide. Figures 2 and 3 show the activity for E and mE substitution.

Figure 4 shows that when a dG* is replaced by an abasic residue D, then the inhibitory activity is strongly reduced.

Figure 5 shows the effect when dT nucleotides are replaced by 5-iododeoxyuridin (JU). Surprisingly the replacement of the dT nucleotides by 5-iododeoxyuridin at the 3' end (ODN 23655) enhanced the inhibitory activity of the derivative.

The result of the first round of the screening steps are summarized in Figure 6. In this Figure 6 the modified bases are shown. In the left column the modified nucleotides used are described in more detail.

**Example 2**

A second round of screening has been performed using the methodology as described above. The oligonucleotides had two substitutions and the sequences are provided in Figure 7.

The results of the second round of screening are summarized in Figure 8. It has been found that 5-methyl-C (dZ) combined with 7-deza dG (dE) or with 2'-OMe-G (mE) substitution increases the potency of the inhibitory activity. This can be observed with oligonucleotide 25077. Furthermore, a deletion of the 5' dT of the sequence increases the potency of inhibitory activity (comparison 25077 vs. 25064).

Figure 9 shows that the 7-deaza inosin substitution increases the potency of inhibition to (see in particular 25080).

Figure 10 shows the effect of a combination of 5-methyl-dC (dZ) and 7-deza-dG (dE) without the 5' dT. Compound having the designation 25069 yields the most efficient inhibitory ODN.

Figure 11 shows the inhibitory effect of short ODNs. 5-Me-C and deaza-dG/dI increases the potency on TLR9. Insertion of deaza-dG/dI results in ODN with increased potency on TLR9. Short inhibitory ODNs inhibit only TLR9, but not TLR7. The 5' extension as shown in compound 25088 also increases potency when combined with DI replacement.

As conclusion from example 2 the effect of modifications in the relevant area can be summarized as follows:

- All ODN containing a 5-Methyl-C immediate 5' to G stretch show improved activity on hLTR9 and mTLR9 cell lines
- Most potent base exchanges at G1 position of G stretch are:
  - 7-deaza-dG (dE)
  - 7-deaza-2'OMe-G (mE)
  - deaza-dI (DI)
- Deletion of 5' dT further improves potency

The best candidates from the second round of experiments are summarized in Figure 12.

Figure 13 shows the surprising effect of a strongly increased potency of inhibition for a JU substitution immediate 3' of the GGG stretch. This can be seen from the biological activity of compound 106219.

Figure 14 shows the surprising results obtained with JU substitutions at other positions of the sequence. This result was not expected.

**Example 3**

In a third round of screening further mutations have been tested. The oligonucleotides contained mainly triple substitutions as shown in Figure 15.

As shown in Figure 16 an unexpected enhancement of the inhibition of activity was observed when three replacements were combined which include the replacement of a dA by 5-lodo-U 3' of the G stretch.

Figure 17 shows that using 5-Bromo dC is well tolerated instead of 5-me dC.

Figure 18 shows that replacing 5-me dC by 5-Octadienyl-dC (ODC) is well tolerated with regard to the inhibition of activity.

Figure 19 shows the effect when 5-Methyl-LNA-C is substituted for 5-methyl-dC.

Figure 20 shows data for 5-Bromo-dC and to 5-Methyl-dC modified analogs.

**Example 4:**

Results were obtained in a test as described above. For determining the immune stimulatory activity of the INH-ODN, no agonist (10103) is added. However, the agonist 10103 is used as positive control in a separate vial.

Data showing that the unmodified parent ODN of modified strong antagonists do not stimulate TLR9 were summarized in Fig. 21A, Fig. 21 B and Fig. 21C.

| Figure | Unmodified parent | Modified Antagonist | Agonist positive control |
|---|---|---|---|
| 21A | 2088 | 25064 | 10103 |
| 21B | 106941 | 106919 | 10103 |
| 21C | 21158 | 25069 | 10103 |

Figure 21 D shows that the TLR9 inhibitory activity of the unmodified parent ODN 2088 and 2114 is independent of the CG dinucleotide motif in ODN 2088, since replacement of C by A resulting in a AG dinucleotide motif has no significant impact on the inhibitory activity.

Figure 21 E shows that ODN 2114 has no TLR9 immune stimulatory activity.

**Example 5:**

Cytokine secretion inhibition assay in B-cells or in plasmacytoid dendritic cells (pDC)

B-cells or pDCs are stimulated with the agonists CpG-ODN 1826 (TLR9) or imiquimod (TLR7) in the presence of 10-fold titrated amounts of INH-ODNs (0.001-10 $\mu$M). As medium, RPMI or DMEM supplemented with 10% FCS and 50 $\mu$M 2-mercaptoethanol is used. After 24 hrs to 72 hors, cytokine levels are determined in the supernatant of culture in 96 well microtiterplates using standard methods (ELISA, multiplex bead array).

**Example 6:**

Determination of inhibitory activity *in vivo* (Inhibition of cytokine/chemokine secretion).

Mice are treated subcutaneously, intramuscular, mucosal, intraperitoneally or intranasally with INH-ODNs and subsequently subcutaneously challenged with the stimulatory ODN 1826 (TLR9), poly-IC (TLR3) or imiquimod (TLR7). Three hours after challenge with the TLR agonist, mice are sacrificed and serum was prepared. Cytokine levels, such as IL-12p40 or IP-10 are determined by standard methods (ELISA, multiplex bead array).

**Example 7:**

Collagen antibody induced and collagen-induced models of rheumatoid arthritis (e.g. as described by Makino et al. J. Nippon Moed Sch 2012, 79, 129-138).

Mice are injected ip with antibodies against murine type II collagen. Three days later, mice received 50 μg of LPS ip. The TLR antagonists are injected s.c., i.p. or i.n. at a dose of 0.1-25mg/kg each day or each second day for 5-7 days. After the treatment, mice are sacrificed and joint swelling is evaluated. Total RNA of affected joints is prepared and level of inflammatory mRNAs (e.g. NLPR3, AIM2, IL-1ß, TNF-a,) in reference to housekeeping genes is determined by RT-PCR. In addition, serum levels of inflammatory cytokines and chemokines can be determined using standard methods.Treatment of the mice with antagonists will reduce the levels of inflammatory cytokines and proteins significantly.

TLR antagonist will also work in a similar model, where rheumatoid arthritis is induced by immunization of the mice with bovine or chicken type II collagen (tail injection, 200 μg) in the presence of complete Freud's adjuvant (CFA). Treatment of the mice with TLR antagonist will reduce the inhibition of the TLR3/7 and 9 induced cytokine production responsible for the onset of the disease.

**Example 8:**

The Imiquimod induced Psoriasis as described in the literature (Roller et al. J Immunol. 2012, 189, 4612-4620) was used.

Mice are shaved and aldara cream (containing 5% imiquimod, 70-75 mg) is applied for at least 5 consecutive days. TLR antagonists are given daily (or each second day, s.c., i.p., i.n. at a dose of 0.1-25mg/kg) after first day of treatment. The mice are evaluated daily for erythema, scalin and hardness of skin. In other experiments, back skin samples are stained with H&E and evaluated histologically.

**Example 9:**

A mouse lupus model using (NZB x NZW)F1 mice was used.

Treatment of (NZB x NZW) F1 mice is started at the onset of the disease (4 months of age) when about 25% of the mice begin to show proteinuria. Mice are treated by s.c. injections of INH-ODN (at a dose of 0.1-25mg/kg) twice a week for five months. After five months treatment (9 months of age), proteinuria and autoantibody levels are measured. One month later, anti-dsDNA antibodies are determined and kidneys are evaluated for IgG deposits using histology. INH-ODN treatment reduces anti-dsDNA antibodies and IgG deposits in the kidney of the lupus mice. Proteinurea and glomerulonephritis are reduced in lupus model mice treated with INH-ODN, resulting in increased survival of INH-ODN treated mice.

**Example 10:**

MRL-*Fas*(*lpr*) mouse lupus model.

MRL-*Fas(lpr)* mice are treated with INH-ODN twice per week using s.c. or i.p. injections or intranasally over 10 weeks and sacrificed at ~3 days after the last dose. Serum samples are taken every two weeks and examined for anti-dsDNA antibodies, for test article serum concentrations. At the end of the study (week 10), kidneys are frozen in OCT medium, and kidney sections are examined for IgG deposits by immunohistochemistry. Urine is collected bi-weekly and tested for protein. INH-ODN treatment reduces anti-dsDNA antibodies and IgG deposits in the kidney of the lupus mice.

**Example 11:**

Delay of onset of diabetes development in 8.3-NOD mice by INH-ODN

The 8.3-NOD mice (age of 3 to 4 weeks) are injected 3x weekly with INH-ODN or controls (PBS or non-stimulatory and non-inhibitory ODN) at a dose of 0.1 mg to 25 mg/kg). Mice are followed for 6 weeks to determine the rate of spontaneous onset of diabetes. Female mice treated with INH-ODN show a delayed diabetes onset compared with the mice injected with PBS or non-stimulatory ODN. Similarly, mice treated with chloroquine (e.g. at 10 mg/kg i.p. daily) show delayed onset of diabetes. Therefore, endogenous TLR7/TLR9 activation contributes to the onset of diabetes in 8.3-NOD mice.

## Claims

1. Inhibitory oligonucleotide having the general formula:

$$X_1C\ C\ N_1N_2\ N_3\ X_2N_4N_5\ G\ G\ G\ N_6\ X_3N_7 \qquad (I)$$

where

C is cytidine or a derivative thereof, whereby the cytidine derivative is selected from the group consisting of 5-methylcytidine, a cytidine-like nucleotide having a chemical modification involving the cytosine base, cytidine nucleoside sugar, or both the cytosine base and the cytidine nucleoside sugar, 2'-O-methylcytidine, 5-bromocytidine, 5-hydroxycytidine, ribocytidine and cytosine-β-D-arabinofuranoside,
G is guanosine or a derivative thereof, whereby the guanosine derivative is selected from the group consisting of 7-deazaguanosine, a guanosine-like nucleotide having a chemical modification involving the guanine base, the guanosine nucleoside sugar or both the guanine base and the guanosine nucleoside sugar,
$X_1$ and $X_3$ is any nucleotide sequence with 0 to 12 bases and each nucleotide is independent of any other, $X_2$ is any nucleotide sequence having 0 to 3 nucleotides,
$N_1$, $N_2$ and $N_3$ are each independently any nucleotide,
$N_4$ and $N_7$ is a pyrimidine or a modified pyrimidine,
$N_5$ is a purin or a modified purin,
$N_6$ is a modified pyrimidine, A or a modified purin,
wherein at least two of the nucleotides $N_4$, $N_5$, $N_6$ or $N_7$ are modified purins or modified pyrimidines.

2. Inhibitory oligonucleotide according to claim 1 having the general formula:

$$X_1C\ C\ N_1N_2\ N_3\ X_2N_4N_5\ G\ G\ G\ N_6\ X_3N_7 \qquad (I)$$

where

C is cytidine or a derivative thereof as defined in claim 1,
G is guanosine or a derivative thereof as defined in claim 1,
$X_1$ and $X_3$ is any nucleotide sequence with 0 to 6 bases and each nucleotide is independent of any other, $X_2$ is 0 or 1 nucleotide,
$N_1$, $N_2$ and $N_3$ are each independently any nucleotide,
$N_4$ and $N_7$ is a pyrimidine or a modified pyrimidine,
$N_5$ is a purin or a modified purin,
$N_6$ is a modified pyrimidine, A or a modified purin,
wherein at least two of the nucleotides $N_4$, $N_5$, $N_6$ or $N_7$ are modified purins or modified pyrimidines, and whereby the oligonucleotide comprises up to or less than 20 nucleosides.

3. Inhibitory oligonucleotide according to claims 1 or 2 having the general formula

$$N_1C\ C\ T\ G\ G\ py\ pu\ G\ G\ G\ px\ A\ G\ py \qquad (II)$$

wherein

C is cytidine or a derivative thereof as defined in claim 1,
G is guanosine or a derivative thereof as defined in claim 1,
$N_1$ is any nucleotide or no nucleotide,
py is a pyrimidine or a modified pyrimidine nucleotide,
pu is a purin or a modified purin nucleotide,
px is a modified pyrimidine, A or a modified purin,
wherein at least two of the nucleotides py, pu and px are modified purins or modified pyrimidines selected from the group consisting of 7-deaza-desoxyguanosine, 7-deaza-2'-O-methylguanosine, inosine, diaminopurin, 6-thio-desoxyguanosine, 6-O-methyl-desoxyguanosine, 7-deaza-inosine, 7-deaza-7-iododesoxyguanosine, 7-aminopropargyldesoxaguanosine, 2-fluoro-cytosine, 5-methylcytosine.

4. Inhibitory oligonucleotide according to claim 3 having the general formula

$N_1$C C T G G py pu G G G (III)

wherein C, G, $N_1$, py, and pu have the meaning as defined in any of the preceding claims.

**5.** Inhibitory oligonucleotide according to claim 3, wherein

Py is 5-substituted cytidine, selected from the group consisting of 5-methyl-dC, 5-bromo-dC and 5-octadienyl-dC, Pu is a 7-deaza purin derivative, selected from the group consisting of 7-deaza-dG, 7-deaza-2'-O-methyl-G, inosine and 7-deaza-inosine and Px is dA or 5-iodo-dU.

**6.** Inhibitory oligonucleotide according to claim 1, **characterized in that** it has the sequence dC*dC*dT*dG*dG*dZ*mE*dG*dG*dG*dA*dA*dG*dT.

**7.** Inhibitory oligonucleotide according to claim 1, **characterized in that** it has the sequence dC*dC*dT*dG*dG*BC*dE*dG*dG*dG*JU*dA*dG*dT.

**8.** Inhibitory oligonucleotide having the general formula IV

$X_1$ A A T G G py pu G G G px A G py (IV)

wherein

Py is 5-substituted cytidine, selected from the group consisting of 5-methyl-dC, 5-bromo-dC and 5-octadienyl-dC, Pu is a 7-deaza purin derivative, selected from the group consisting of 7-deaza-dG, 7-deaza-2'-O-methyl-G, inosine and 7-deaza-inosine and Px is dA or 5-iodo-dU.
Px is dA, 5-substituted deoxyuridine, and 5-iodo-uridine and $X_1$ is any nucleotide or no nucleotide.

**9.** Inhibitory oligonucleotide according to any of the claims 1-8 for use as a TLR antagonist with strongly enhanced potency.

**10.** Inhibitory oligonucleotide according to any of claims 1-9 for use in the prevention and/or treatment of cancer, an autoimmune disorder, airway inflammation, inflammatory disorders, infectious diseases, skin disorders, allergy, asthma or a disease caused by a pathogen.

**11.** Pharmaceutical composition comprising at least one inhibitory oligonucleotide according to any one of claims 1-9.

**12.** Pharmaceutical composition according to claim 11 comprising further at least one additive and/or carrier.

**13.** Pharmaceutical composition according to claim 11 or 12 for use in the treatment of cancer, an autoimmune disorder, airway inflammation, inflammatory disorders, infectious disease, skin disorders, allergy, asthma or a disease caused by a pathogen.

| SEQ ID NO. | CPG Lot | OEX Code | Description | Length |
|---|---|---|---|---|
| 1 | 2088 | dT*dC*dC*dT*dG*dG*dC*dG*dG*dG*dG*dA*dA*dG*dT | | 15 |
| 2 | 21595 | dT*dC*dC*dT*dG*dG*dC*dE*dG*dG*dG*dA*dA*dG*dT | 7-deaza-dG | 15 |
| 3 | 20844 | dT*dC*dC*dT*dG*dG*dC*dG*dE*dG*dG*dA*dA*dG*dT | 7-deaza-dG | 15 |
| 4 | 21596 | dT*dC*dC*dT*dG*dG*dC*dG*dG*dE*dG*dA*dA*dG*dT | 7-deaza-dG | 15 |
| 5 | 21597 | dT*dC*dC*dT*dG*dG*dC*dG*dG*dG*dE*dA*dA*dG*dT | 7-deaza-dG | 15 |
| 6 | 24888 | dT*dC*dC*dT*dG*dG*dC*mE*dG*dG*dG*dA*dA*dG*dT | 7-deaza-2'-O-methyl-G | 15 |
| 7 | 24889 | dT*dC*dC*dT*dG*dG*dC*dG*mE*dG*dG*dA*dA*dG*dT | 7-deaza-2'-O-methyl-G | 15 |
| 8 | 2489 | dT*dC*dC*dT*dG*dG*dC*dG*dG*mE*dG*dA*dA*dG*dT | 7-deaza-2'-O-methyl-G | 15 |
| 9 | 24891 | dT*dC*dC*dT*dG*dG*dC*dG*dG*dG*mE*dA*dA*dG*dT | 7-deaza-2'-O-methyl-G | 15 |
| 10 | 24892 | dT*dC*dC*dT*dG*dG*dC*I*dG*dG*dG*dA*dA*dG*dT | Inosine | 15 |
| 11 | 24893 | dT*dC*dC*dT*dG*dG*dC*dG*I*dG*dG*dA*dA*dG*dT | Inosine | 15 |
| 12 | 24894 | dT*dC*dC*dT*dG*dG*dC*dG*dG*I*dG*dA*dA*dG*dT | Inosine | 15 |
| 13 | 24895 | dT*dC*dC*dT*dG*dG*dC*dG*dG*dG*I*dA*dA*dG*dT | Inosine | 15 |
| 14 | 24896 | dT*dC*dC*dT*dG*dG*dC*V*dG*dG*dG*dA*dA*dG*dT | 2,6-Diaminopurine | 15 |
| 15 | 21598 | dT*dC*dC*dT*dG*dG*dC*dG*V*dG*dG*dA*dA*dG*dT | 2,6-Diaminopurine | 15 |
| 16 | 24897 | dT*dC*dC*dT*dG*dG*dC*dG*dG*V*dG*dA*dA*dG*dT | 2,6-Diaminopurine | 15 |
| 17 | 24898 | dT*dC*dC*dT*dG*dG*dC*dG*dG*dG*V*dA*dA*dG*dT | 2,6-Diaminopurine | 15 |
| 18 | 24899 | dT*dC*dC*dT*dG*dG*dC*8G*dG*dG*dG*dA*dA*dG*dT | 7-deaza-8-aza-dG | 15 |
| 19 | 23291 | dT*dC*dC*dT*dG*dG*dC*dG*8G*dG*dG*dA*dA*dG*dT | 7-deaza-8-aza-dG | 15 |
| 20 | 24900 | dT*dC*dC*dT*dG*dG*dC*dG*dG*8G*dG*dA*dA*dG*dT | 7-deaza-8-aza-dG | 15 |
| 21 | 24901 | dT*dC*dC*dT*dG*dG*dC*dG*dG*dG*8G*dA*dA*dG*dT | 7-deaza-8-aza-dG | 15 |
| 22 | 24902 | dT*dC*dC*dT*dG*dG*dC*O*dG*dG*dG*dA*dA*dG*dT | 8-Oxo-dG | 15 |
| 23 | 24904 | dT*dC*dC*dT*dG*dG*dC*dG*dG*O*dG*dA*dA*dG*dT | 8-Oxo-dG | 15 |
| 24 | 24905 | dT*dC*dC*dT*dG*dG*dC*dG*dG*dG*O*dA*dA*dG*dT | 8-Oxo-dG | 15 |
| 25 | 24906 | dT*dC*dC*dT*dG*dG*dC*Q*dG*dG*dG*dA*dA*dG*dT | 8-Oxo-dA | 15 |
| 26 | 24907 | dT*dC*dC*dT*dG*dG*dC*dG*Q*dG*dG*dA*dA*dG*dT | 8-Oxo-dA | 15 |
| 27 | 24908 | dT*dC*dC*dT*dG*dG*dC*dG*dG*Q*dG*dA*dA*dG*dT | 8-Oxo-dA | 15 |

Fig. 1 A

| SEQ ID NO. | CPG Lot | OEX Code | Description | Length |
|---|---|---|---|---|
| 28 | 24909 | dT*dC*dC*dT*dG*dG*dC*dG*dG*dG*Q*dA*dA*dG*dT | 8-Oxo-dA | 15 |
| 29 | 24910 | dT*dC*dC*dT*dG*dG*dC*6TG*dG*dG*dG*dA*dA*dG*dT | 6-Thio-dG | 15 |
| 30 | 24911 | dT*dC*dC*dT*dG*dG*dC*dG*6TG*dG*dG*dA*dA*dG*dT | 6-Thio-dG | 15 |
| 31 | 24912 | dT*dC*dC*dT*dG*dG*dC*dG*dG*6TG*dG*dA*dA*dG*dT | 6-Thio-dG | 15 |
| 32 | 24913 | dT*dC*dC*dT*dG*dG*dC*dG*dG*dG*6TG*dA*dA*dG*dT | 6-Thio-dG | 15 |
| 33 | 24914 | dT*dC*dC*dT*dG*dG*dC*6MG*dG*dG*dG*dA*dA*dG*dT | 6-O-methyl-dG | 15 |
| 34 | 24915 | dT*dC*dC*dT*dG*dG*dC*dG*6MG*dG*dG*dA*dA*dG*dT | 6-O-methyl-dG | 15 |
| 35 | 24917 | dT*dC*dC*dT*dG*dG*dC*dG*dG*dG*6MG*dA*dA*dG*dT | 6-O-methyl-dG | 15 |
| 36 | 24918 | dT*dC*dC*dT*dG*dG*dC*DI*dG*dG*dG*dA*dA*dG*dT | 7-deaza inosin | 15 |
| 37 | 24919 | dT*dC*dC*dT*dG*dG*dC*dG*DI*dG*dG*dA*dA*dG*dT | 7-deaza inosin | 15 |
| 38 | 24920 | dT*dC*dC*dT*dG*dG*dC*dG*dG*DI*dG*dA*dA*dG*dT | 7-deaza inosin | 15 |
| 39 | 24921 | dT*dC*dC*dT*dG*dG*dC*dG*dG*dG*DI*dA*dA*dG*dT | 7-deaza inosin | 15 |
| 40 | 24922 | dT*dC*dC*dT*dG*dG*dC*JG*dG*dG*dG*dA*dA*dG*dT | 7-deaza 7-iodo dG | 15 |
| 41 | 24923 | dT*dC*dC*dT*dG*dG*dC*dG*JG*dG*dG*dA*dA*dG*dT | 7-deaza 7-iodo dG | 15 |
| 42 | 24924 | dT*dC*dC*dT*dG*dG*dC*dG*dG*JG*dG*dA*dA*dG*dT | 7-deaza 7-iodo dG | 15 |
| 43 | 24925 | dT*dC*dC*dT*dG*dG*dC*dG*dG*dG*JG*dA*dA*dG*dT | 7-deaza 7-iodo dG | 15 |
| 44 | 24926 | dT*dC*dC*dT*dG*dG*dC*PG*dG*dG*dG*dA*dA*dG*dT | 7-aminopropargyl dG | 15 |
| 45 | 24927 | dT*dC*dC*dT*dG*dG*dC*dG*PG*dG*dG*dA*dA*dG*dT | 7-aminopropargyl dG | 15 |
| 46 | 24928 | dT*dC*dC*dT*dG*dG*dC*dG*dG*PG*dG*dA*dA*dG*dT | 7-aminopropargyl dG | 15 |
| 47 | 24929 | dT*dC*dC*dT*dG*dG*dC*dG*dG*dG*PG*dA*dA*dG*dT | 7-aminopropargyl dG | 15 |
| 48 | 24930 | dT*dC*dC*dT*dG*dG*dC*dA*dG*dG*dG*dA*dA*dG*dT | G, A base change | 15 |
| 49 | 20955 | dT*dC*dC*dT*dG*dG*dC*dG*dG*dA*dG*dA*dA*dG*dT | G, A base change | 15 |
| 50 | 20954 | dT*dC*dC*dT*dG*dG*dC*dG*dG*dG*dA*dA*dA*dG*dT | G, A base change | 15 |
| 51 | 24933 | dT*dC*dC*dT*dG*dG*dC*dG*dG*dA*dG*dA*dG*dG*dT | G, A base change | 15 |
| 52 | 24934 | dT*dC*dC*dT*dG*dG*dC*dG*dG*dG*dA*dA*dG*dG*dT | G, A base change | 15 |
| 53 | 24935 | dT*dC*dC*dT*dG*dG*dC*dT*dG*dG*dG*dA*dA*dG*dT | G, T base change | 15 |
| 54 | 24936 | dT*dC*dC*dT*dG*dG*dC*dG*dT*dG*dG*dA*dA*dG*dT | G, T base change | 15 |
| 55 | 24937 | dT*dC*dC*dT*dG*dG*dC*dG*dG*dT*dG*dA*dA*dG*dT | G, T base change | 15 |

**Fig. 1 B**

| SEQ ID NO. | CPG Lot | OEX Code | Description | Length |
|---|---|---|---|---|
| 56 | 24938 | dT*dC*dC*dT*dG*dG*dC*dG*dG*dG*dT*dA*dA*dG*dT | G, T base change | 15 |
| 57 | 24939 | dT*dC*dC*dT*dG*dG*dC*dZ*dG*dG*dG*dA*dA*dG*dT | G, 5-methyl-C base change () | 15 |
| 58 | 24940 | dT*dC*dC*dT*dG*dG*dC*dG*dZ*dG*dG*dA*dA*dG*dT | G, 5-methyl-C base change | 15 |
| 59 | 24941 | dT*dC*dC*dT*dG*dG*dC*dG*dG*dZ*dG*dA*dA*dG*dT | G, 5-methyl-C base change | 15 |
| 60 | 24942 | dT*dC*dC*dT*dG*dG*dC*dG*dG*dG*dZ*dA*dA*dG*dT | G, 5-methyl-C base change | 15 |
| 61 | 24943 | dT*dC*dC*dT*dG*dG*dC*dU*dG*dG*dG*dA*dA*dG*dT | G, U base change | 15 |
| 62 | 24944 | dT*dC*dC*dT*dG*dG*dC*dG*dU*dG*dG*dA*dA*dG*dT | G, U base change | 15 |
| 63 | 24945 | dT*dC*dC*dT*dG*dG*dC*dG*dG*dU*dG*dA*dA*dG*dT | G, U base change | 15 |
| 64 | 24946 | dT*dC*dC*dT*dG*dG*dC*dG*dG*dG*dU*dA*dA*dG*dT | G, U base change | 15 |
| 65 | 24947 | dT*dC*dC*dT*dG*dG*dC*D*dG*dG*dG*dA*dA*dG*dT | G to D change (dSpacer, abasic) | 15 |
| 66 | 24948 | dT*dC*dC*dT*dG*dG*dC*dG*D*dG*dG*dA*dA*dG*dT | G to D change | 15 |
| 67 | 24949 | dT*dC*dC*dT*dG*dG*dC*dG*dG*D*dG*dA*dA*dG*dT | G to D change | 15 |
| 68 | 24950 | dT*dC*dC*dT*dG*dG*dC*dG*dG*dG*D*dA*dA*dG*dT | G to D change | 15 |
| 69 | 24951 | dT*dC*dC*dT*dG*dG*dC*J*dG*dG*dG*dA*dA*dG*dT | G to J change (C3 spacer) | 15 |
| 70 | 24952 | dT*dC*dC*dT*dG*dG*dC*dG*J*dG*dG*dA*dA*dG*dT | G to J change | 15 |
| 71 | 24953 | dT*dC*dC*dT*dG*dG*dC*dG*dG*J*dG*dA*dA*dG*dT | G to J change | 15 |
| 72 | 24954 | dT*dC*dC*dT*dG*dG*dC*dG*dG*dG*J*dA*dA*dG*dT | G to J change | 15 |
| 73 | 24955 | dT*dC*dC*dT*dG*dG*dC*dG*D*dG*dG*dG*dA*dA*dG*dT | dSpacer insertion | 16 |
| 74 | 24956 | dT*dC*dC*dT*dG*dG*dC*dG*dG*D*dG*dG*dA*dA*dG*dT | dSpacer insertion | 16 |
| 75 | 24957 | dT*dC*dC*dT*dG*dG*dC*dG*dG*dG*D*dG*dA*dA*dG*dT | dSpacer insertion | 16 |
| 76 | 24958 | dT*dC*dC*dT*dG*dG*dC*dG*J*dG*dG*dG*dA*dA*dG*dT | J (C3 spacer) insertion | 16 |
| 77 | 24959 | dT*dC*dC*dT*dG*dG*dC*dG*dG*J*dG*dG*dA*dA*dG*dT | J insertion | 16 |
| 78 | 24960 | dT*dC*dC*dT*dG*dG*dC*dG*dG*dG*J*dG*dA*dA*dG*dT | J insertion | 16 |
| 79 | 24961 | dT*dC*dC*dT*dG*dG*dC*dG*dA*dG*dG*dG*dA*dA*dG*dT | dA insertion | 16 |
| 80 | 24962 | dT*dC*dC*dT*dG*dG*dC*dG*dG*dA*dG*dG*dA*dA*dG*dT | dA insertion | 16 |
| 81 | 24963 | dT*dC*dC*dT*dG*dG*dC*dG*dG*dG*dA*dG*dA*dA*dG*dT | dA insertion | 16 |
| 82 | 24964 | dT*dC*dC*dT*dG*dG*dC*dG*dE*dG*dG*dG*dA*dA*dG*dT | dE insertion | 16 |

Fig. 1 C

| SEQ ID NO. | CPG Lot | OEX Code | Description | Length |
|---|---|---|---|---|
| 83 | 24965 | dT*dC*dC*dT*dG*dG*dC*dG*dG*dE*dG*dG*dA*dA*dG*dT | dE insertion | 16 |
| 84 | 24966 | dT*dC*dC*dT*dG*dG*dC*dG*dG*dG*dE*dG*dA*dA*dG*dT | dE insertion | 16 |
| 85 | 23487 | JU*dC*dC*dT*dG*dG*dC*dG*dG*dG*dG*dA*dA*dG*dT | T to JU change | 15 |
| 86 | 23488 | dT*dC*dC*JU*dG*dG*dC*dG*dG*dG*dG*dA*dA*dG*dT | T to JU change | 15 |
| 87 | 23489 | JU*dC*dC*JU*dG*dG*dC*dG*dG*dG*dG*dA*dA*dG*dT | 2x (T to JU change) | 15 |
| 88 | 23655 | dT*dC*dC*dT*dG*dG*dC*dG*dG*dG*dG*dA*dA*dG*JU | T to JU change | 15 |
| 89 | 24967 | dT*dC*dC*dT*dG*dG*JU*dG*dG*dG*dG*dA*dA*dG*dT | C to JU change | 15 |
| 90 | 24968 | dT*frC*dC*dT*dG*dG*dC*dG*dG*dG*dG*dA*dA*dG*dT | C to 2'-fluoro-C | 15 |
| 91 | 24969 | dT*dC*frC*dT*dG*dG*dC*dG*dG*dG*dG*dA*dA*dG*dT | C to 2'-fluoro-C | 15 |
| 92 | 24970 | dT*frC*frC*dT*dG*dG*dC*dG*dG*dG*dG*dA*dA*dG*dT | C to 2'-fluoro-C | 15 |
| 93 | 24971 | dT*dC*dC*dT*dG*dG*frC*dG*dG*dG*dG*dA*dA*dG*dT | C to 2'-fluoro-C | 15 |
| 94 | 24972 | dT*faC*dC*dT*dG*dG*dC*dG*dG*dG*dG*dA*dA*dG*dT | C to 2'-FANA-C | 15 |
| 95 | 24973 | dT*dC*faC*dT*dG*dG*dC*dG*dG*dG*dG*dA*dA*dG*dT | C to 2'-FANA-C | 15 |
| 96 | 24974 | dT*faC*faC*dT*dG*dG*dC*dG*dG*dG*dG*dA*dA*dG*dT | C to 2'-FANA-C | 15 |
| 97 | 24975 | dT*dC*dC*dT*dG*dG*faC*dG*dG*dG*dG*dA*dA*dG*dT | C to 2'-FANA-C | 15 |
| 98 | 24976 | dT*dC*dC*dT*dG*dG*dC*dG*dG*dG*dG*mA*mA*mG*dT | 3' purin as 2'-Omethyl | 15 |
| 99 | 24977 | dT*dC*dC*dT*dG*dG*dC*dG*dG*dG*dG*frA*frA*frG*dT | 3' purin as 2'-F | 15 |
| 100 | 24978 | dT*dC*dC*dT*dG*dG*dC*dG*dG*dG*dG*ßA*ßA*ßG*dT | 3' purin as LNA | 15 |
| 101 | 24979 | dT*dC*dC*dT*dG*dG*dZ*dG*dG*dG*dG*dA*dA*dG*dT | C to 5-methyl-C | 15 |
| 102 | 2114 | dT*dC*dC*dT*dG*dG*dA*dG*dG*dG*dG*dA*dA*dG*dT | C to A | 15 |
| 103 | 2420 | dT*dC*dC*dT*dG*dG*dT*dG*dG*dG*dG*dA*dA*dG*dT | C to T | 15 |
| 104 | 24980 | dT*dC*dC*dT*dG*dG*dU*dG*dG*dG*dG*dA*dA*dG*dT | C to U | 15 |
| 105 | 24981 | dT*dC*dC*dT*dG*dG*D*dG*dG*dG*dG*dA*dA*dG*dT | C to D (dSpacer, abasic) | 15 |
| 106 | 24982 | dT*dC*dC*dT*dG*dG*dE*dG*dG*dG*dG*dA*dA*dG*dT | C to 7-deaza-dG (dE) | 15 |
| 107 | 24983 | dT*dC*dC*dT*dG*dG*dE*dG*dE*dG*dG*dA*dA*dG*dT | C to 7-deaza-dG (dE) | 15 |
| 108 | 24984 | dT*dC*dC*dT*dG*dG*dE*dG*dA*dG*dG*dA*dA*dG*dT | C to 7-deaza-dG and G à A | 15 |
| 109 | 24985 | dT*dC*dC*dT*dG*dG*mC*dG*dG*dG*dG*dA*dA*dG*dT | C to 2'-O-methyl-C | 15 |
| 110 | 24986 | dT*dC*dC*dT*dG*dG*ßZ*dG*dG*dG*dG*dA*dA*dG*dT | C to LNA-Z | 15 |
| 111 | 21158 | dC*dC*dT*dG*dG*dC*dG*dG*dG*dG | | 10 |

Fig. 1 D

| SEQ ID NO. | CPG Lot | OEX Code | Description | Length |
|---|---|---|---|---|
| 112 | 24987 | dC*dC*dT*dG*dG*dC*dE*dG*dG*dG | 7-deaza-dG (dE) | 10 |
| 113 | 24988 | dC*dC*dT*dG*dG*dC*dG*dE*dG*dG | 7-deaza-dG | 10 |
| 114 | 24989 | dC*dC*dT*dG*dG*dC*dG*dG*dE*dG | 7-deaza-dG | 10 |
| 115 | 24990 | dC*dC*dT*dG*dG*dC*dG*dG*dG*dE | 7-deaza-dG | 10 |
| 116 | 24991 | dC*dC*dT*dG*dG*dC*mE*dG*dG*dG | 7-deaza-2'-O-methyl-G | 10 |
| 117 | 24992 | dC*dC*dT*dG*dG*dC*dG*mE*dG*dG | 7-deaza-2'-O-methyl-G | 10 |
| 118 | 24993 | dC*dC*dT*dG*dG*dC*dG*dG*mE*dG | 7-deaza-2'-O-methyl-G | 10 |
| 119 | 24994 | dC*dC*dT*dG*dG*dC*dG*dG*dG*mE | 7-deaza-2'-O-methyl-G | 10 |
| 120 | 24995 | dC*dC*dT*dG*dG*dC*dG*I*dG*dG | Deoxy-Inosine (I) | 10 |
| 121 | 24996 | dC*dC*dT*dG*dG*dC*dG*dG*I*dG | Deoxy-Inosine (I) | 10 |
| 122 | 24997 | dC*dC*dT*dG*dG*dC*dG*dG*dG*I | Deoxy-Inosine (I) | 10 |
| 123 | 24998 | dC*dC*dT*dG*dG*dC*dG*6TG*dG*dG | 6-Thio-dG | 10 |
| 124 | 24999 | dC*dC*dT*dG*dG*dC*dG*dG*6TG*dG | 6-Thio-dG | 10 |
| 125 | 25000 | dC*dC*dT*dG*dG*dC*dG*dG*dG*6TG | 6-Thio-dG | 10 |
| 126 | 25001 | dC*dC*dT*dG*dG*dC*dG*DI*dG*dG | 7-deaza deoxyinosine (DI) | 10 |
| 127 | 25002 | dC*dC*dT*dG*dG*dC*dG*dG*DI*dG | 7-deaza deoxyinosine (DI) | 10 |
| 128 | 25003 | dC*dC*dT*dG*dG*dC*dG*dG*dG*DI | 7-deaza deoxyinosine (DI) | 10 |
| 129 | 25007 | dC*dC*dT*dG*dG*dC*dG*dT*dG*dG | G to T | 10 |
| 130 | 25008 | dC*dC*dT*dG*dG*dC*dG*dG*dT*dG | G to T | 10 |
| 131 | 25009 | dC*dC*dT*dG*dG*dC*dG*dG*dG*dT | G to T | 10 |
| 132 | 25010 | dC*dC*dT*dG*dG*dC*dZ*dG*dG*dG | G to Z (5-methyl dC) | 10 |
| 133 | 25011 | dC*dC*dT*dG*dG*dC*dG*dZ*dG*dG | G to Z | 10 |
| 134 | 25012 | dC*dC*dT*dG*dG*dC*dG*dG*dZ*dG | G to Z | 10 |
| 135 | 25013 | dC*dC*dT*dG*dG*dC*dG*dG*dG*dZ | G to Z | 10 |
| 136 | 25014 | dC*dC*dT*dG*dG*dC*dU*dG*dG*dG | G to U | 10 |
| 137 | 25015 | dC*dC*dT*dG*dG*dC*dG*dU*dG*dG | G to U | 10 |
| 138 | 25016 | dC*dC*dT*dG*dG*dC*dG*dG*dU*dG | G to U | 10 |
| 139 | 25017 | dC*dC*dT*dG*dG*dC*dG*dG*dG*dU | G to U | 10 |

Fig. 1 E

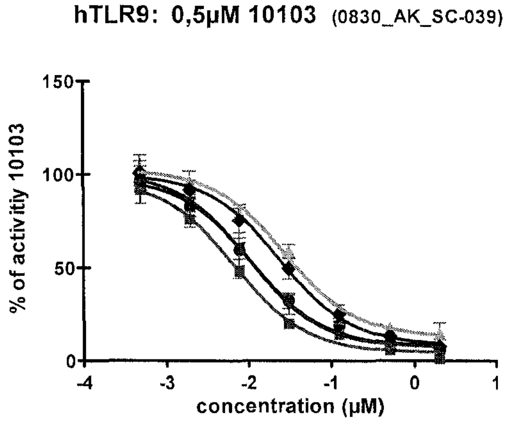


| | |
|---|---|
| ● 2088 | dT*dC*dC*dT*dG*dG*dC*dG*dG*dG*dG*dA*dA*dG*dT |
| ■ 21595 | dT*dC*dC*dT*dG*dG*dC***dE***dG*dG*dG*dA*dA*dG*dT |
| ▲ 20844 | dT*dC*dC*dT*dG*dG*dC*dG***dE***dG*dG*dA*dA*dG*dT |
| ▼ 21596 | dT*dC*dC*dT*dG*dG*dC*dG*dG***dE***dG*dA*dA*dG*dT |
| ♦ 21597 | dT*dC*dC*dT*dG*dG*dC*dG*dG*dG***dE***dA*dA*dG*dT |

Fig. 2

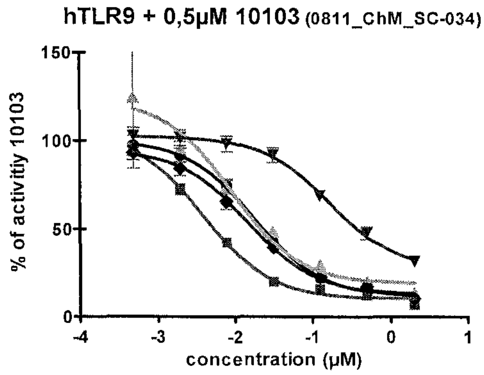


| | |
|---|---|
| ● 2088 | dT*dC*dC*dT*dG*dG*dC*dG*dG*dG*dG*dA*dA*dG*dT |
| ■ 24888 | dT*dC*dC*dT*dG*dG*dC***mE***dG*dG*dG*dA*dA*dG*dT |
| ▲ 24889 | dT*dC*dC*dT*dG*dG*dC*dG***mE***dG*dG*dA*dA*dG*dT |
| ▼ 2489 | dT*dC*dC*dT*dG*dG*dC*dG*dG***mE***dG*dA*dA*dG*dT |
| ♦ 24891 | dT*dC*dC*dT*dG*dG*dC*dG*dG*dG***mE***dA*dA*dG*dT |

Fig. 3

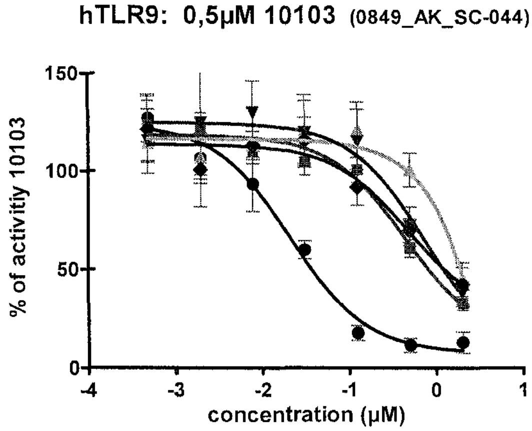


| | |
|---|---|
| ● 2088 | dT*dC*dC*dT*dG*dG*dC*dG*dG*dG*dG*dA*dA*dG*dT |
| ■ 24947 | dT*dC*dC*dT*dG*dG*dC*D*dG*dG*dG*dA*dA*dG*dT |
| ▲ 24948 | dT*dC*dC*dT*dG*dG*dC*dG*D*dG*dG*dA*dA*dG*dT |
| ▼ 24949 | dT*dC*dC*dT*dG*dG*dC*dG*dG*D*dG*dA*dA*dG*dT |
| ♦ 24950 | dT*dC*dC*dT*dG*dG*dC*dG*dG*dG*D*dA*dA*dG*dT |

Fig. 4

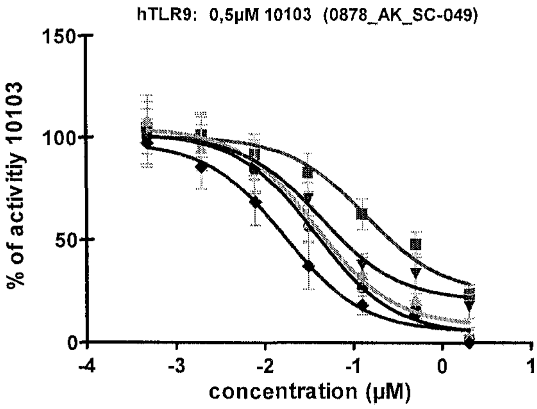


| | |
|---|---|
| ● 2088 | dT*dC*dC*dT*dG*dG*dC*dG*dG*dG*dG*dA*dA*dG*dT |
| ■ 23487 | JU*dC*dC*dT*dG*dG*dC*dG*dG*dG*dG*dA*dA*dG*dT |
| ▲ 23488 | dT*dC*dC*JU*dG*dG*dC*dG*dG*dG*dG*dA*dA*dG*dT |
| ▼ 23489 | JU*dC*dC*JU*dG*dG*dC*dG*dG*dG*dG*dA*dA*dG*dT |
| ♦ 23655 | dT*dC*dC*dT*dG*dG*dC*dG*dG*dG*dG*dA*dA*dG*JU |

Fig. 5

| | | |
|---|---|---|
| **24888** | dT*dC*dC*dT*dG*dG*dC***mE***dG*dG*dG*dA*dA*dG*dT | **7-deaza-2'-O-methyl-G** |
| **24892** | dT*dC*dC*dT*dG*dG*dC***I***dG*dG*dG*dA*dA*dG*dT | **Inosin** |
| **24918** | dT*dC*dC*dT*dG*dG*dC***DI***dG*dG*dG*dA*dA*dG*dT | **7-deaza inosin** |
| **24930** | dT*dC*dC*dT*dG*dG*dC***dA***dG*dG*dG*dA*dA*dG*dT | **G -->A** |
| **24935** | dT*dC*dC*dT*dG*dG*dC***dT***dG*dG*dG*dA*dA*dG*dT | **G -->T** |
| **24943** | dT*dC*dC*dT*dG*dG*dC***dU***dG*dG*dG*dA*dA*dG*dT | **G -->U** |
| **24945** | dT*dC*dC*dT*dG*dG*dC*dG*dG***dU***dG*dA*dA*dG*dT | **G -->U** |
| **24972** | dT***faC***dC*dT*dG*dG*dC*dG*dG*dG*dG*dA*dA*dG*dT | **C --> 2'-FANA-C** |
| **24979** | dT*dC*dC*dT*dG*dG***dZ***dG*dG*dG*dG*dA*dA*dG*dT | **C --> 5-methyl-dC** |
| **24987** | dC*dC*dT*dG*dG*dC***dE***dG*dG*dG | **7-deaza-dG** |
| **24991** | dC*dC*dT*dG*dG*dC***mE***dG*dG*dG | **7-deaza-2'-O-methyl-G** |
| **25028** | dC*dC*dT*dG*dG*dC***DI***dG*dG*dG | **7-deaza inosin** |
| **25006** | dC*dC*dT*dG*dG*dC***dT***dG*dG*dG | **G -->T** |

Fig. 6

| SEQ ID NO. | CPG Lot | OEX Code (new) | Description | Length |
|---|---|---|---|---|
| 140 | 25064 | dT*dC*dC*dT*dG*dG*dZ*dE*dG*dG*dG*dA*dA*dG*dT | dZ-substitution, 7-deaza-dG (dE) | 15 |
| 141 | 25065 | dT*dC*dC*dT*dG*dG*dZ*mE*dG*dG*dG*dA*dA*dG*dT | dZ-substitution, 7-deaza-2'-O-methyl-G | 15 |
| 142 | 25066 | dT*dC*dC*dT*dG*dG*dZ*I*dG*dG*dG*dA*dA*dG*dT | dZ-substitution, deoxy-inosin | 15 |
| 143 | 25067 | dT*dC*dC*dT*dG*dG*dZ*DI*dG*dG*dG*dA*dA*dG*dT | dZ-substitution, 7-deaza deoxy-inosin | 15 |
| 144 | 25068 | dT*dC*dC*dT*dG*dG*dZ*dT*dG*dG*dG*dA*dA*dG*dT | dZ-substitution, GàT | 15 |
| 145 | 25069 | dC*dC*dT*dG*dG*dZ*dE*dG*dG*dG | dZ-substitution, 7-deaza-dG | 10 |
| 146 | 25070 | dC*dC*dT*dG*dG*dZ*mE*dG*dG*dG | dZ-substitution, 7-deaza-2'-O-methyl-G | 10 |
| 147 | 25071 | dC*dC*dT*dG*dG*dZ*DI*dG*dG*dG | dZ-substitution, 7-deaza inosin | 10 |
| 148 | 25072 | dC*dC*dT*dG*dG*dZ*dT*dG*dG*dG | dZ-substitution, G to T | 10 |
| 149 | 25073 | dT*dC*dC*dT*dG*dG*dC*dE*dG*dG*dG*mA*mA*mG*dT | 2´-OMe-substitution,, 7-deaza-dG | 15 |
| 150 | 25074 | dT*dC*dC*dT*dG*dG*dC*mE*dG*dG*dG*mA*mA*mG*dT | 2´-OMe-substitution, 7-deaza-2'-O-methyl-G | 15 |
| 151 | 25075 | dT*dC*dC*dT*dG*dG*dC*I*dG*dG*dG*mA*mA*mG*dT | 2´-OMe-substitution, Deoxy-Inosin | 15 |
| 152 | 25076 | dT*dC*dC*dT*dG*dG*dC*DI*dG*dG*dG*mA*mA*mG*dT | 2´-OMe-substitution, 7-deaza deoxyinosin | 15 |
| 153 | 25077 | dC*dC*dT*dG*dG*dZ*dE*dG*dG*dG*dA*dA*dG*dT | 5´-T deletion, 7-deaza-dG | 14 |
| 154 | 25078 | dC*dC*dT*dG*dG*dZ*mE*dG*dG*dG*dA*dA*dG*dT | 5´-T deletion, 7-deaza-2'-O-methyl-G | 14 |
| 155 | 25079 | dC*dC*dT*dG*dG*dZ*I*dG*dG*dG*dA*dA*dG*dT | 5´-T deletion, deoxyinosin | 14 |
| 156 | 25080 | dC*dC*dT*dG*dG*dZ*DI*dG*dG*dG*dA*dA*dG*dT | 5´-T deletion, 7-deaza deoxyinosin | 14 |
| 157 | 25081 | dC*dT*dC*dC*dT*dG*dG*dC*dE*dG*dG*dG*dA*dA*dG*dT | 5´-extension, 7-deaza-dG | 16 |
| 158 | 25082 | dC*dT*dC*dC*dT*dG*dG*dC*mE*dG*dG*dG*dA*dA*dG*dT | 5´-extension, 7-deaza-2'-O-methyl-G | 16 |
| 159 | 25083 | dC*dT*dC*dC*dT*dG*dG*dC*I*dG*dG*dG*dA*dA*dG*dT | 5´-extension, deoxyinosin | 16 |
| 160 | 25084 | dC*dT*dC*dC*dT*dG*dG*dC*DI*dG*dG*dG*dA*dA*dG*dT | 5´-extension, 7-deaza deoxyinosin | 16 |
| 161 | 25085 | dT*dG*dC*dT*dC*dC*dT*dG*dG*dC*dE*dG*dG*dG*dA*dA*dG*dT | 5´-extension, 7-deaza-dG | 18 |
| 162 | 25086 | dT*dG*dC*dT*dC*dC*dT*dG*dG*dC*mE*dG*dG*dG*dA*dA*dG*dT | 5´-extension, 7-deaza-2'-O-methyl-G | 18 |

Fig. 7 A

| SEQ ID NO. | CPG Lot | OEX Code (new) | Description | Length |
|---|---|---|---|---|
| 163 | 25087 | dT*dG*dC*dT*dC*dC*dT*dG*dG*dC*I*dG*dG*dG*dA*dA*dG*dT | 5´-extension: deoxyinosin | 18 |
| 164 | 25088 | dT*dG*dC*dT*dC*dC*dT*dG*dG*dC*DI*dG*dG*dG*dA*dA*dG*dT | 5´-extension: 7-deaza deoxyinosin | 18 |
| 165 | 25089 | dT*dT*dT*dT*dC*dC*dT*dG*dG*dC*dE*dG*dG*dG*dA*dA*dG*dT | 5´-extension: 7-deaza-dG | 18 |
| 166 | 25090 | dT*dT*dT*dT*dC*dC*dT*dG*dG*dC*mE*dG*dG*dG*dA*dA*dG*dT | 5´-extension: 7-deaza-2'-O-methyl-G | 18 |
| 167 | 25091 | dT*dT*dT*dT*dC*dC*dT*dG*dG*dC*I*dG*dG*dG*dA*dA*dG*dT | 5´-extension: deoxyinosin | 18 |
| 168 | 25092 | dT*dT*dT*dT*dC*dC*dT*dG*dG*dC*DI*dG*dG*dG*dA*dA*dG*dT | 5´-extension: 7-deaza deoxyinosin | 18 |
| 169 | 25093 | dC*dT*dC*dC*dT*dG*dG*dC*dE*dG*dG*dG*dA*dA*dG*dT*dT*dT*dT | 3´-5´-extension: 7-deaza-dG | 19 |
| 170 | 25094 | dC*dT*dC*dC*dT*dG*dG*dC*mE*dG*dG*dG*dA*dA*dG*dT*dT*dT*d T | 3´-5´-extension: 7-deaza-2'-O-methyl-G | 19 |
| 171 | 25095 | dC*dT*dC*dC*dT*dG*dG*dC*I*dG*dG*dG*dA*dA*dG*dT*dT*dT*dT | 3´-5´-extension: deoxyinosin | 19 |
| 172 | 25096 | dC*dT*dC*dC*dT*dG*dG*dC*DI*dG*dG*dG*dA*dA*dG*dT*dT*dT*dT | 3´-5´-extension: 7-deaza deoxyinosin | 19 |
| 173 | 25111 | dC*dT*dC*dC*dC*dG*dC*dG*dC*dG*dC*dG*dG*dG | palindrome with motif | 14 |

Fig. 7 B

**hTLR9** + **0,5µM 10103** (0939_AK_SC-055)

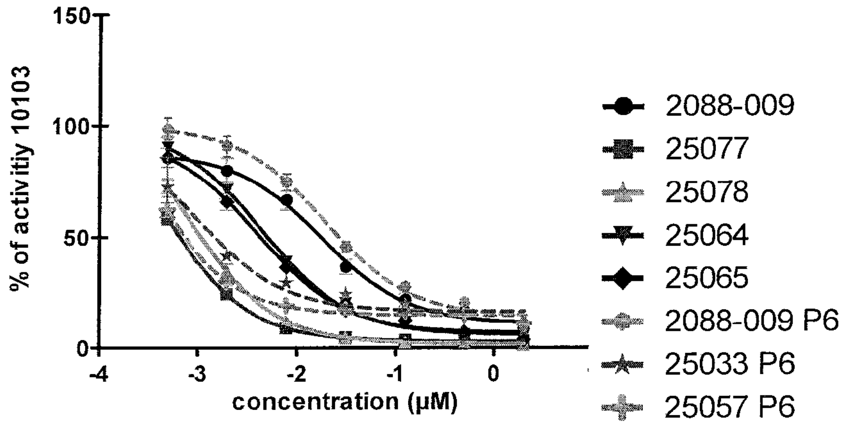


| | | |
|---|---|---|
| **2088** | dT*dC*dC*dT*dG*dG*dC*dG*dG*dG*dG*dA*dA*dG*dT | |
| **25077** | dC*dC*dT*dG*dG**dZ*dE***dG*dG*dG*dA*dA*dG*dT | 5´-T deletion: 7-deaza-dG |
| **25078** | dC*dC*dT*dG*dG**dZ*mE***dG*dG*dG*dA*dA*dG*dT | 5´-T deletion: 7-deaza-2'-O-methyl-G |
| **25064** | dT*dC*dC*dT*dG*dG**dZ*dE***dG*dG*dG*dA*dA*dG*dT | Z-substitution: 7-deaza-dG |
| **25065** | dT*dC*dC*dT*dG*dG**dZ*mE***dG*dG*dG*dA*dA*dG*dT | Z-substitution: 7-deaza-2'-O-methyl-G |

| | | |
|---|---|---|
| **2088** | dT*dC*dC*dT*dG*dG*dC*dG*dG*dG*dG*dA*dA*dG*dT | |
| **25033** | dT*dG*dC*dT*dC*dC*dT*dG*dG*dA*dG*dG*dG*dG*dT*dT*dG*dT | IRS 954 |
| **25057** | dC*dC*dT*dG*dG*dA*dT*dG*dG*dG*dA*dA*dC*dT*dT*dA*dC*dC*dG*dC*dT*dG*dC*dA | Lenert, INH-18 |

Fig. 8

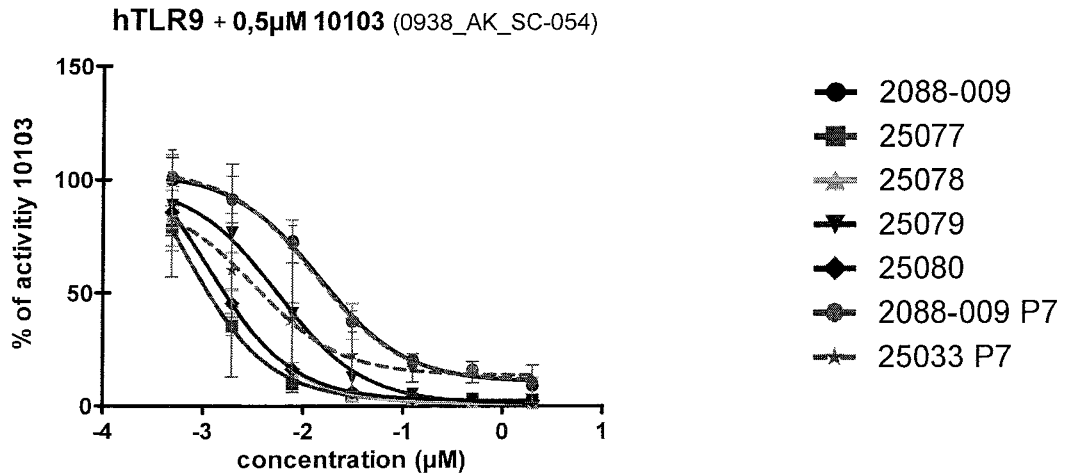


| | | |
|---|---|---|
| **2088** | dT*dC*dC*dT*dG*dG*dC*dG*dG*dG*dG*dA*dA*dG*dT | |
| **25077** | dC*dC*dT*dG*dG***dZ*dE***dG*dG*dG*dA*dA*dG*dT | 5´-T deletion: 7-deaza-dG |
| **25078** | dC*dC*dT*dG*dG***dZ*mE***dG*dG*dG*dA*dA*dG*dT | 5´-T deletion: 7-deaza-2'-O-methyl-G |
| **25079** | dC*dC*dT*dG*dG***dZ* I***dG*dG*dG*dA*dA*dG*dT | 5´-T deletion: Inosin |
| **25080** | dC*dC*dT*dG*dG***dZ*DI***dG*dG*dG*dA*dA*dG*dT | 5´-T deletion: 7-deaza inosin |

| | | |
|---|---|---|
| **2088** | dT*dC*dC*dT*dG*dG*dC*dG*dG*dG*dG*dA*dA*dG*dT | |
| **25033** | dT*dG*dC*dT*dC*dC*dT*dG*dG*dA*dG*dG*dG*dG*dT*dT*dG*dT | IRS 954 |
| **25057** | dC*dC*dT*dG*dG*dA*dT*dG*dG*dG*dA*dA*dC*dT*dT*dA*dC*dC*dG*dC*dT*dG*dC*dA | Lenert, INH-18 |

Fig. 9

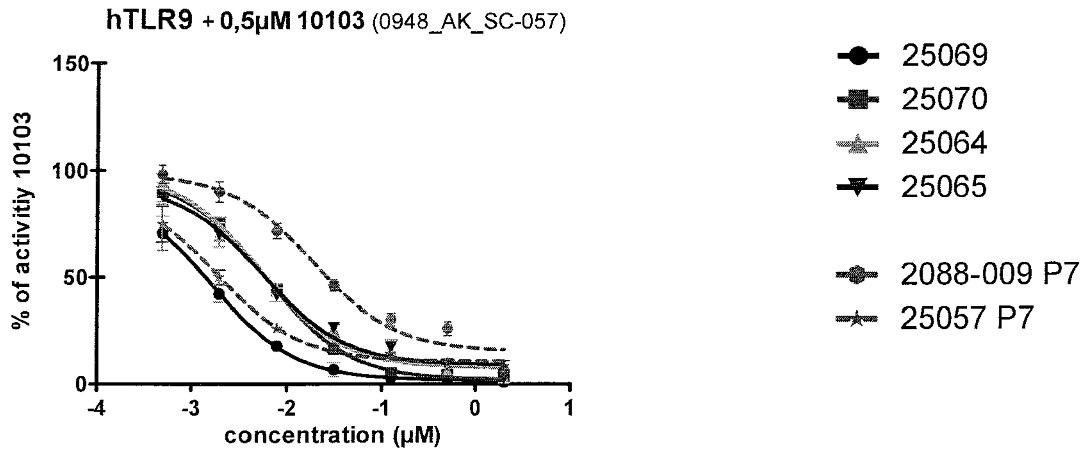


| | | |
|---|---|---|
| 2088 | dT*dC*dC*dT*dG*dG*dC*dG*dG*dG*dG*dA*dA*dG*dT | |
| 25069 | dC*dC*dT*dG*dG***dZ***dE***dG*dG*dG | Z-substitution: 7-deaza-dG |
| 25070 | dC*dC*dT*dG*dG***dZ***mE***dG*dG*dG | methyl-G |
| 25064 | dT*dC*dC*dT*dG*dG***dZ***dE***dG*dG*dG*dA*dA*dG*dT | Z-substitution: 7-deaza-dG |
| 25065 | dT*dC*dC*dT*dG*dG***dZ***mE***dG*dG*dG*dA*dA*dG*dT | methyl-G |

| | | |
|---|---|---|
| 2088 | dT*dC*dC*dT*dG*dG*dC*dG*dG*dG*dG*dA*dA*dG*dT | |
| 25033 | dT*dG*dC*dT*dC*dC*dT*dG*dG*dA*dG*dG*dG*dG*dT*dT*dG*dT | IRS 954 |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 |

Fig. 10

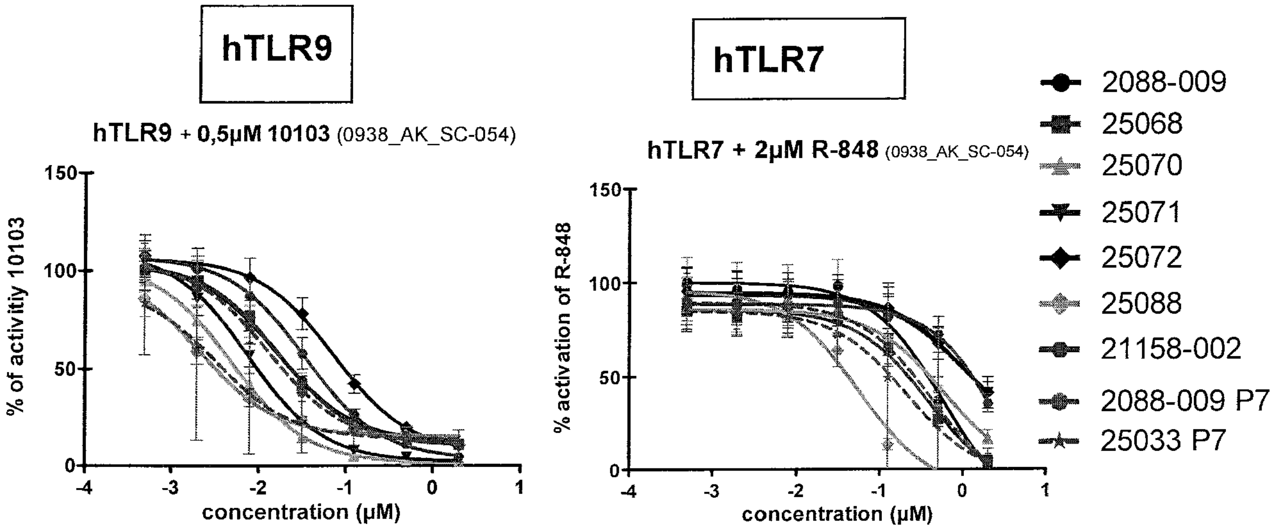


| | | |
|---|---|---|
| 2088 | dT*dC*dC*dT*dG*dG*dC*dG*dG*dG*dG*dA*dA*dG*dT | |
| 25068 | dT*dC*dC*dT*dG*dG***dZ*dT***dG*dG*dG*dA*dA*dG*dT | Z-substitution: GàT |
| 25070 | dC*dC*dT*dG*dG***dZ*mE***dG*dG*dG | Z-substitution: 7-deaza-2'-O-methyl-G |
| 25071 | dC*dC*dT*dG*dG***dZ*DI***dG*dG*dG | Z-substitution: 7-deaza inosin |
| 25072 | dC*dC*dT*dG*dG***dZ*dT***dG*dG*dG | Z-substitution: GàT |
| 25088 | dT*dG*dC*dT*dC*dC*dT*dG*dG*dC***DI***dG*dG*dG*dA*dA*dG*dT | 5'-extension: 7-deaza inosin |
| 21158 | dC*dC*dT*dG*dG*dC*dG*dG*dG*dG | |

| | | |
|---|---|---|
| 2088 | dT*dC*dC*dT*dG*dG*dC*dG*dG*dG*dG*dA*dA*dG*dT | |
| 25033 | dT*dG*dC*dT*dC*dC*dT*dG*dG*dA*dG*dG*dG*dG*dT*dT*dG*dT | IRS 954 |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 |

Fig. 11

| | | | hTLR9 | mTLR9 | hTLR7 | hTLR8 |
|---|---|---|---|---|---|---|
| 25077 | dC*dC*dT*dG*dG*dZ*dE*dG*dG*dG*dA*dA*dG*dT | 5´-T deletion: 7-deaza-dG | +++ | +++ | +++ | +++ |
| 25078 | dC*dC*dT*dG*dG*dZ*mE*dG*dG*dG*dA*dA*dG*dT | G | +++ | +++ | +++ | +++ |
| 25069 | dC*dC*dT*dG*dG*dZ*dE*dG*dG*dG | Z-substitution: 7-deaza-dG | +++ | +++ | - | - |
| 25070 | dC*dC*dT*dG*dG*dZ*mE*dG*dG*dG | G | +++ | +++ | - | - |
| 25080 | dC*dC*dT*dG*dG*dZ*DI*dG*dG*dG*dA*dA*dG*dT | 5´-T deletion: 7-deaza inosin | +++ | +++ | +++ | +++ |
| | | | | | | |
| 25071 | dC*dC*dT*dG*dG*dZ*DI*dG*dG*dG | Z-substitution: 7-deaza inosin | ++ | ++ | - | - |
| 25067 | dT*dC*dC*dT*dG*dG*dZ*DI*dG*dG*dG*dA*dA*dG*dT | Z-substitution: 7-deaza inosin | ++ | ++ | +++ | +++ |
| 25068 | dT*dC*dC*dT*dG*dG*dZ*dT*dG*dG*dG*dA*dA*dG*dT | Z-substitution: GàT | ++ | ++ | +++ | +++ |
| 25064 | dT*dC*dC*dT*dG*dG*dZ*dE*dG*dG*dG*dA*dA*dG*dT | Z-substitution: 7-deaza-dG | ++ | ++ | +++ | +++ |
| 25065 | dT*dC*dC*dT*dG*dG*dZ*mE*dG*dG*dG*dA*dA*dG*dT | G | ++ | ++ | +++ | +++ |
| 2088 | dT*dC*dC*dT*dG*dG*dC*dG*dG*dG*dG*dA*dA*dG*dT | | ++ | ++ | +++ | +++ |
| | | | | | | |
| 25033 | dT*dG*dC*dT*dC*dC*dT*dG*dG*dA*dG*dG*dG*dG*dT*dT*dG*dT | IRS 954 | ++ | ++ | +++ | +++ |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 | +++ | +++ | +++ | !!!! (enhancement of activity) |

Fig. 12

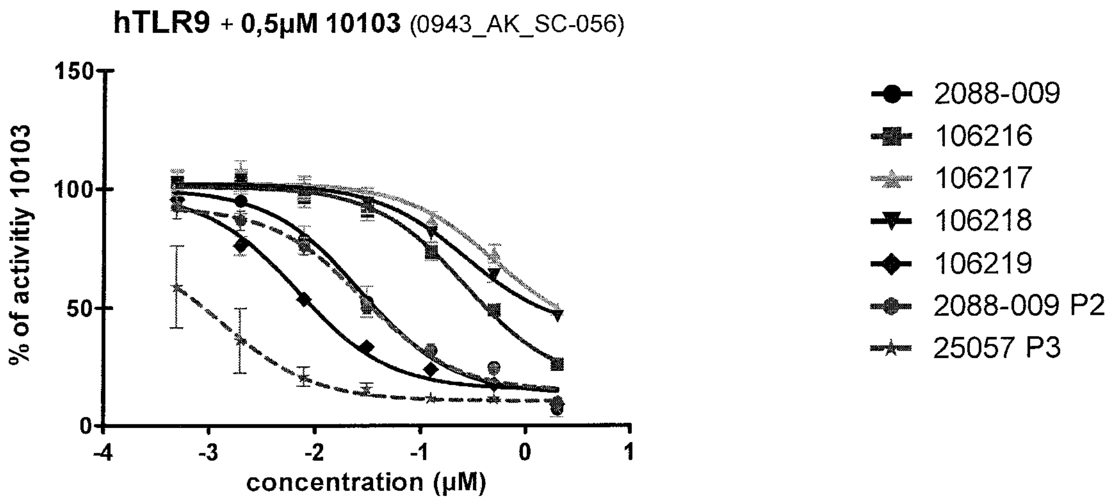


| | | |
|---|---|---|
| 106216 | dT*dC*dC*dT*dG*dG*dC*dG*JU*dG*dG*dA*dA*dG*dT | 5-Iodo-dU derivatives of S-class oligonucleotides |
| 106217 | dT*dC*dC*dT*dG*dG*dC*dG*dG*JU*dG*dA*dA*dG*dT | 5-Iodo-dU derivatives of S-class oligonucleotides |
| 106218 | dT*dC*dC*dT*dG*dG*dC*dG*dG*dG*JU*dA*dA*dG*dT | 5-Iodo-dU derivatives of S-class oligonucleotides |
| 106219 | dT*dC*dC*dT*dG*dG*dC*dG*dG*dG*dG*JU*dA*dG*dT | 5-Iodo-dU derivatives of S-class oligonucleotides |
| | | |
| 2088 | dT*dC*dC*dT*dG*dG*dC*dG*dG*dG*dG*dA*dA*dG*dT | |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 |

Fig. 13

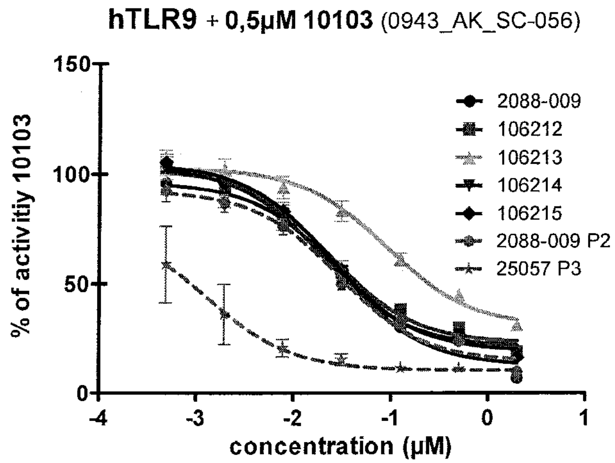


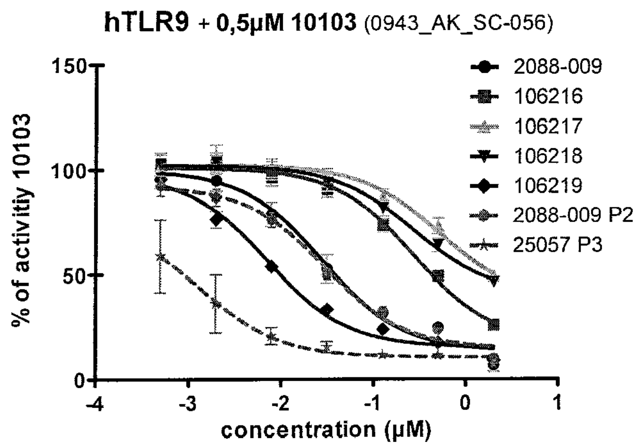


| | | |
|---|---|---|
| 106212 | dT*dC*dC*dT*JU*dG*dC*dG*dG*dG*dG*dA*dA*dG*dT | 5-Iodo-dU derivatives of S-class oligonucleotides |
| 106213 | dT*dC*dC*dT*dG*JU*dC*dG*dG*dG*dG*dA*dA*dG*dT | 5-Iodo-dU derivatives of S-class oligonucleotides |
| 106214 | dT*dC*dC*dT*dG*dG*JU*dG*dG*dG*dG*dA*dA*dG*dT | 5-Iodo-dU derivatives of S-class oligonucleotides |
| 106215 | dT*dC*dC*dT*dG*dG*dC*JU*dG*dG*dG*dA*dA*dG*dT | 5-Iodo-dU derivatives of S-class oligonucleotides |

| | | |
|---|---|---|
| 2088 | dT*dC*dC*dT*dG*dG*dC*dG*dG*dG*dG*dA*dA*dG*dT | |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 |

| | | |
|---|---|---|
| 106216 | dT*dC*dC*dT*dG*dG*dC*dG*JU*dG*dG*dA*dA*dG*dT | 5-Iodo-dU derivatives of S-class oligonucleotides |
| 106217 | dT*dC*dC*dT*dG*dG*dC*dG*dG*JU*dG*dA*dA*dG*dT | 5-Iodo-dU derivatives of S-class oligonucleotides |
| 106218 | dT*dC*dC*dT*dG*dG*dC*dG*dG*dG*JU*dA*dA*dG*dT | 5-Iodo-dU derivatives of S-class oligonucleotides |
| 106219 | dT*dC*dC*dT*dG*dG*dC*dG*dG*dG*dG*JU*dA*dG*dT | 5-Iodo-dU derivatives of S-class oligonucleotides |

| | | |
|---|---|---|
| 2088 | dT*dC*dC*dT*dG*dG*dC*dG*dG*dG*dG*dA*dA*dG*dT | |
| 25057 | dC*dC*dT*dG*dG*dA*dT*dG*dG*dG*dA*dA*dC*dT*dT*dA*dC*dC*dG*dC*dT*dG*dC*dA | Lenert, INH-18 |

Fig.14

| SEQ ID NO. | CPG Lot | OEX Code | Description | Length |
|---|---|---|---|---|
| 174 | 106918-001 | dC*dC*dT*dG*dG*dZ*dE*dG*dG*dG*dA*dA*dG*dT | -- | 14 |
| 175 | 106919-001 | dC*dC*dT*dG*dG*dZ*dE*dG*dG*dG*JU*dA*dG*dT | -- | 14 |
| 176 | 106920-001 | dC*dC*dT*dG*dG*dZ*dG*dG*dG*dG*JU*dA*dG*dT | -- | 14 |
| 177 | 106921-001 | dC*dC*dT*dG*dG*dC*dE*dG*dG*dG*JU*dA*dG*dT | -- | 14 |
| 178 | 106922-001 | dC*dC*dT*dG*dG*dC*dG*dG*dG*dG*JU*dA*dG*dT | -- | 14 |
| 179 | 106923-001 | dC*dC*dT*dG*dG*PC*dE*dG*dG*dG*dA*dA*dG*dT | 5 Propinyl-C | 14 |
| 180 | 106924-001 | dC*dC*dT*dG*dG*PC*dE*dG*dG*dG*JU*dA*dG*dT | 5 Propinyl-C | 14 |
| 181 | 106925-001 | dC*dC*dT*dG*dG*PC*dG*dG*dG*dG*JU*dA*dG*dT | 5 Propinyl-C | 14 |
| 182 | 106926-001 | dC*dC*dT*dG*dG*ODC*dE*dG*dG*dG*dA*dA*dG*dT | 5-Octadienyl-dC | 14 |
| 183 | 106927-001 | dC*dC*dT*dG*dG*ODC*dE*dG*dG*dG*JU*dA*dG*dT | 5-Octadienyl-dC | 14 |
| 184 | 106928-001 | dC*dC*dT*dG*dG*ODC*dG*dG*dG*dG*JU*dA*dG*dT | 5-Octadienyl-dC | 14 |
| 185 | 106929-001 | dC*dC*dT*dG*dG*BC*dE*dG*dG*dG*dA*dA*dG*dT | 5-Bromo-dC | 14 |
| 186 | 106930-001 | dC*dC*dT*dG*dG*BC*dE*dG*dG*dG*JU*dA*dG*dT | 5-Bromo-dC | 14 |
| 187 | 106931-001 | dC*dC*dT*dG*dG*BC*dG*dG*dG*dG*JU*dA*dG*dT | 5-Bromo-dC | 14 |
| 188 | 106932-001 | dC*dC*dT*dG*dG*JC*dE*dG*dG*dG*dA*dA*dG*dT | 5-Iodo-dC | 14 |
| 189 | 106933-001 | dC*dC*dT*dG*dG*JC*dE*dG*dG*dG*JU*dA*dG*dT | 5-Iodo-dC | 14 |
| 190 | 106934-001 | dC*dC*dT*dG*dG*JC*dG*dG*dG*dG*JU*dA*dG*dT | 5-Iodo-dC | 14 |
| 191 | 106935-001 | dC*dC*dT*dG*dG*ßZ*dE*dG*dG*dG*dA*dA*dG*dT | LNA 5-Me-C | 14 |
| 192 | 106936-001 | dC*dC*dT*dG*dG*ßZ*dE*dG*dG*dG*JU*dA*dG*dT | LNA 5-Me-C | 14 |
| 193 | 106937-001 | dC*dC*dT*dG*dG*ßZ*dG*dG*dG*dG*JU*dA*dG*dT | LNA 5-Me-C | 14 |
| 194 | 106938-001 | dC*dC*dT*dG*dG*dZ*dG*dG*dG*dG*dA*dA*dG*dT | dZ | 14 |
| 195 | 106939-001 | dC*dC*dT*dG*dG*dZ*dG*dT*dG*dG*dA*dA*dG*dT | dZ | 14 |
| 196 | 106940-001 | dC*dT*dT*dG*dG*dZ*dG*dG*dG*dG*dA*dA*dG*dT | dZ | 14 |
| 197 | 106941-001 | dC*dC*dT*dG*dG*dC*dG*dG*dG*dG*dA*dA*dG*dT | 2088 5'N-1 | 14 |
| 198 | 106942-001 | dC*dC*dT*dG*dG*dC*dE*dG*dG*dG*dA*dA*dG*dT | 21595 5'-N-1 | 14 |
| 199 | 106943-001 | dT*dG*dC*dT*dC*dC*dT*dG*dG*dA*dE*dG*dG*dG*dT*dT*dG*dT | 7-deaza | 18 |

Fig. 15

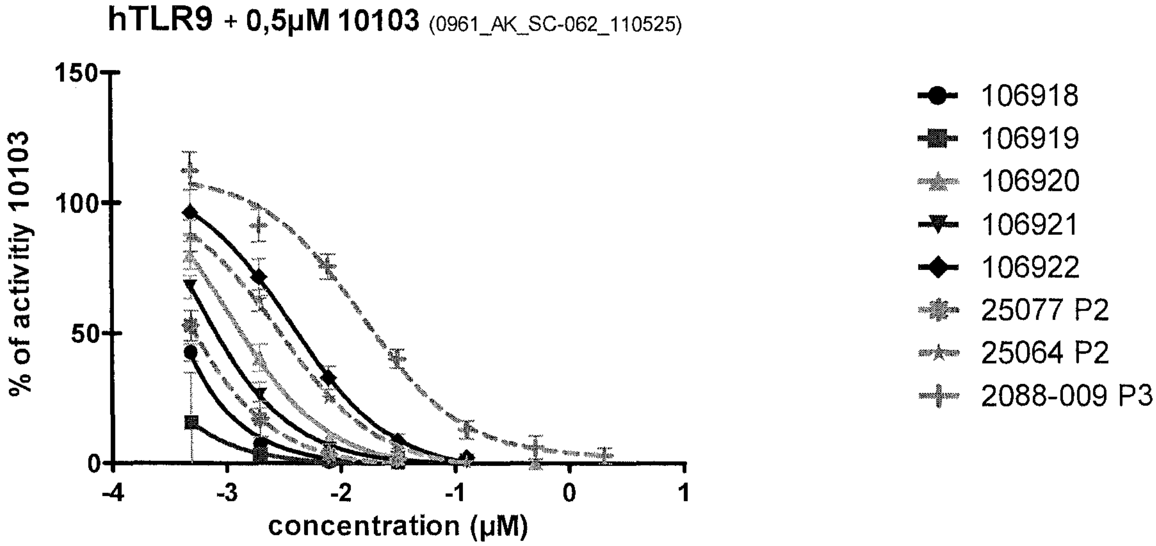


| | |
|---|---|
| 106918-001 | dC*dC*dT*dG*dG*dZ*dE*dG*dG*dG*dA*dA*dG*dT |
| 106919-001 | dC*dC*dT*dG*dG*dZ*dE*dG*dG*dG*JU*dA*dG*dT |
| 106920-001 | dC*dC*dT*dG*dG*dZ*dG*dG*dG*dG*JU*dA*dG*dT |
| 106921-001 | dC*dC*dT*dG*dG*dC*dE*dG*dG*dG*JU*dA*dG*dT |
| 106922-001 | dC*dC*dT*dG*dG*dC*dG*dG*dG*dG*JU*dA*dG*dT |

| | |
|---|---|
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |
| 2088 | dT*dC*dC*dT*dG*dG*dC*dG*dG*dG*dG*dA*dA*dG*dT |

Fig. 16

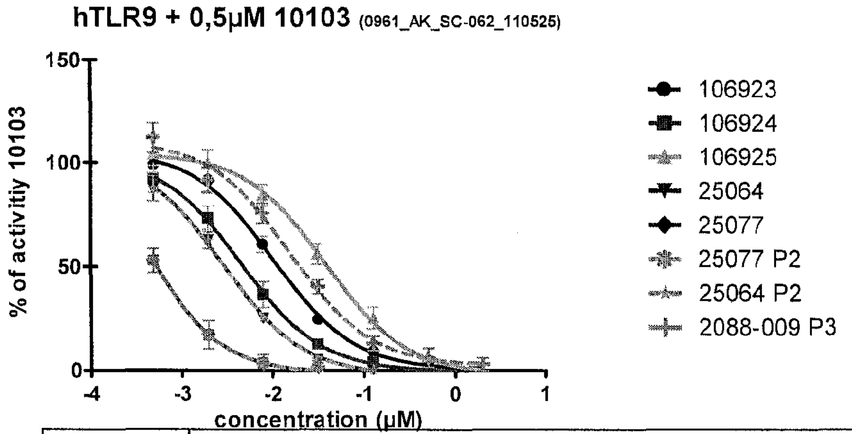


| | |
|---|---|
| 106923-001 | dC*dC*dT*dG*dG*PC*dE*dG*dG*dG*dA*dA*dG*dT |
| 106924-001 | dC*dC*dT*dG*dG*PC*dE*dG*dG*dG*JU*dA*dG*dT |
| 106925-001 | dC*dC*dT*dG*dG*PC*dG*dG*dG*dG*JU*dA*dG*dT |
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |

| | |
|---|---|
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |
| 2088 | dT*dC*dC*dT*dG*dG*dC*dG*dG*dG*dG*dA*dA*dG*dT |

Fig. 17

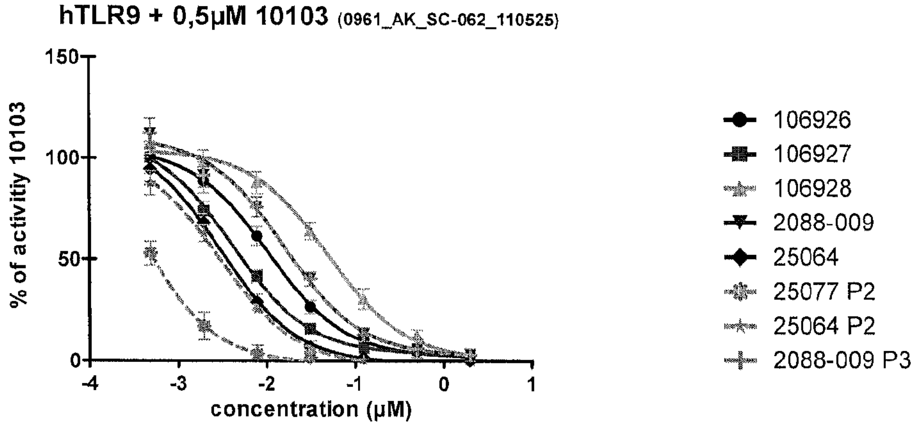


| | |
|---|---|
| 106926-001 | dC*dC*dT*dG*dG*ODC*dE*dG*dG*dG*dA*dA*dG*dT |
| 106927-001 | dC*dC*dT*dG*dG*ODC*dE*dG*dG*dG*JU*dA*dG*dT |
| 106928-001 | dC*dC*dT*dG*dG*ODC*dG*dG*dG*dG*JU*dA*dG*dT |
| 2088 | dT*dC*dC*dT*dG*dG*dC*dG*dG*dG*dG*dA*dA*dG*dT |
| 25064 | dT*dC*dC*dT*dG*dG*dZ*dE*dG*dG*dG*dA*dA*dG*dT |
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |
| 2088 | dT*dC*dC*dT*dG*dG*dC*dG*dG*dG*dG*dA*dA*dG*dT |

Fig. 18

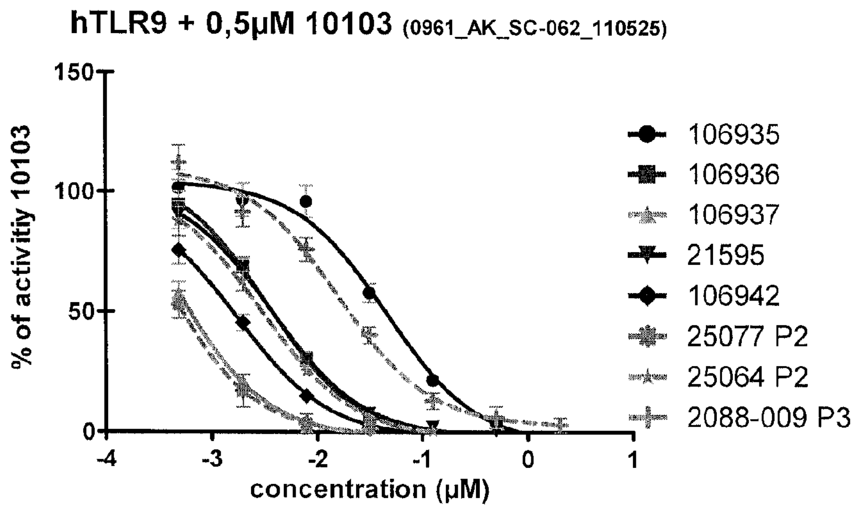


| | |
|---|---|
| 106935-001 | dC*dC*dT*dG*dG*ßZ*dE*dG*dG*dG*dA*dA*dG*dT |
| 106936-001 | dC*dC*dT*dG*dG*ßZ*dE*dG*dG*dG*JU*dA*dG*dT |
| 106937-001 | dC*dC*dT*dG*dG*ßZ*dG*dG*dG*dG*JU*dA*dG*dT |
| 21595 | dT*dC*dC*dT*dG*dG*dC*dE*dG*dG*dG*dA*dA*dG*dT |
| 106942-001 | dC*dC*dT*dG*dG*dC*dE*dG*dG*dG*dA*dA*dG*dT |

| | |
|---|---|
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |
| 2088 | dT*dC*dC*dT*dG*dG*dC*dG*dG*dG*dG*dA*dA*dG*dT |

Fig. 19

5-Bromo-dC similar to 5-Methyl-dC

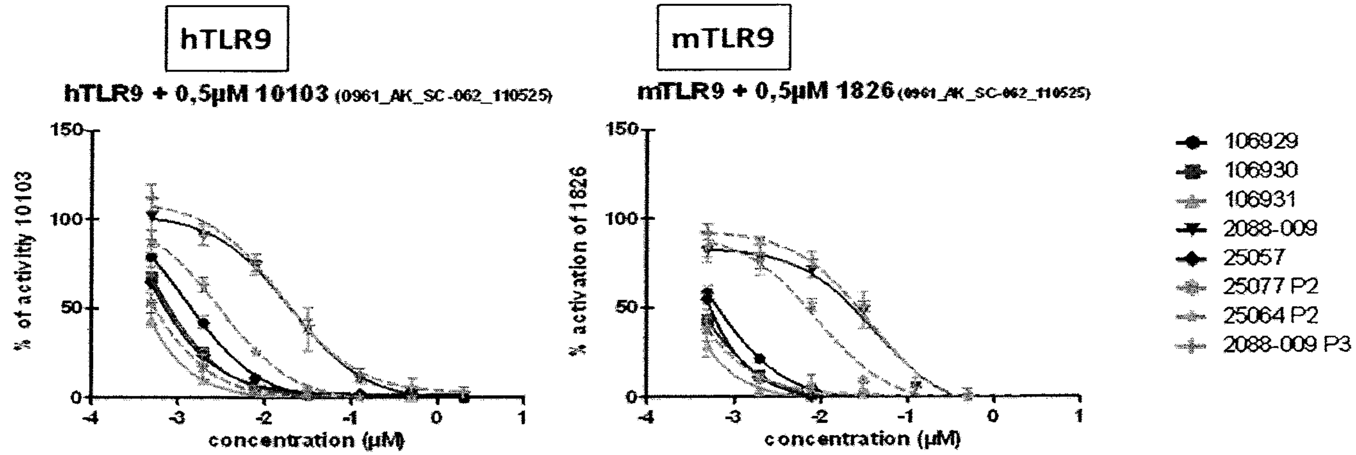


| | |
|---|---|
| 106929-001 | dC*dC*dT*dG*dG*BC*dE*dG*dG*dG*dA*dA*dG*dT |
| 106930-001 | dC*dC*dT*dG*dG*BC*dE*dG*dG*dG*JU*dA*dG*dT |
| 106931-001 | dC*dC*dT*dG*dG*BC*dG*dG*dG*dG*JU*dA*dG*dT |
| 2088 | dT*dC*dC*dT*dG*dG*dC*dG*dG*dG*dG*dA*dA*dG*dT |
| 25057 | dC*dC*dT*dG*dG*dA*dT*dG*dG*dG*dA*dA*dC*dT*dT*dA*dC*dC*dG*dC*dT*dG*dC*dA |

| | |
|---|---|
| 25077 | dC*dC*dT*dG*dG*dZ*dE*dG*dG*dG*dA*dA*dG*dT |
| 25064 | dT*dC*dC*dT*dG*dG*dZ*dE*dG*dG*dG*dA*dA*dG*dT |
| 2088 | dT*dC*dC*dT*dG*dG*dC*dG*dG*dG*dG*dA*dA*dG*dT |

1

Figure 20

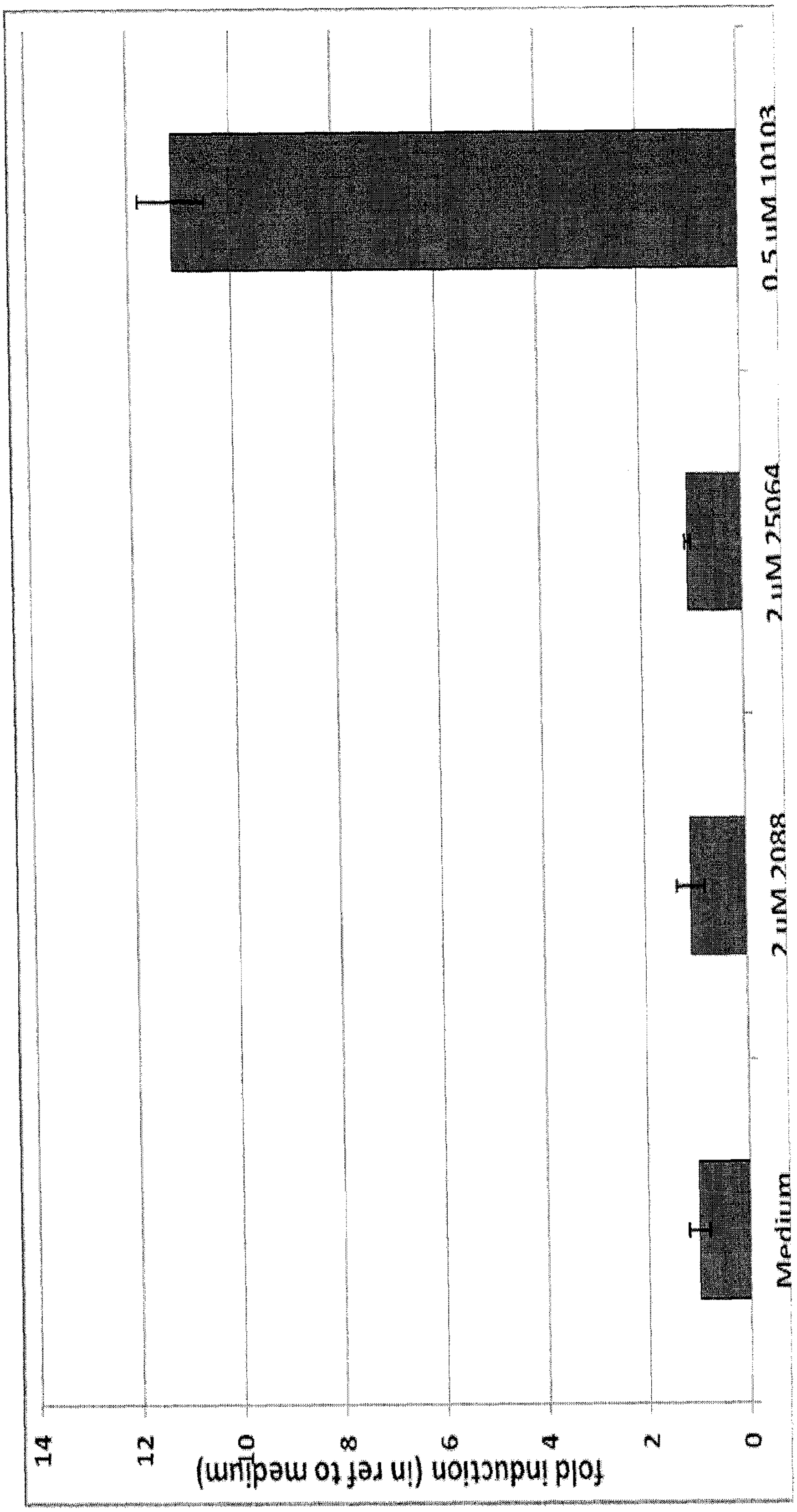
fold induction (in ref to medium)
0
2
4
6
8
10
12
14
Medium
2 uM 2088
2 uM 25064
0.5 uM 10103

Figure 21A

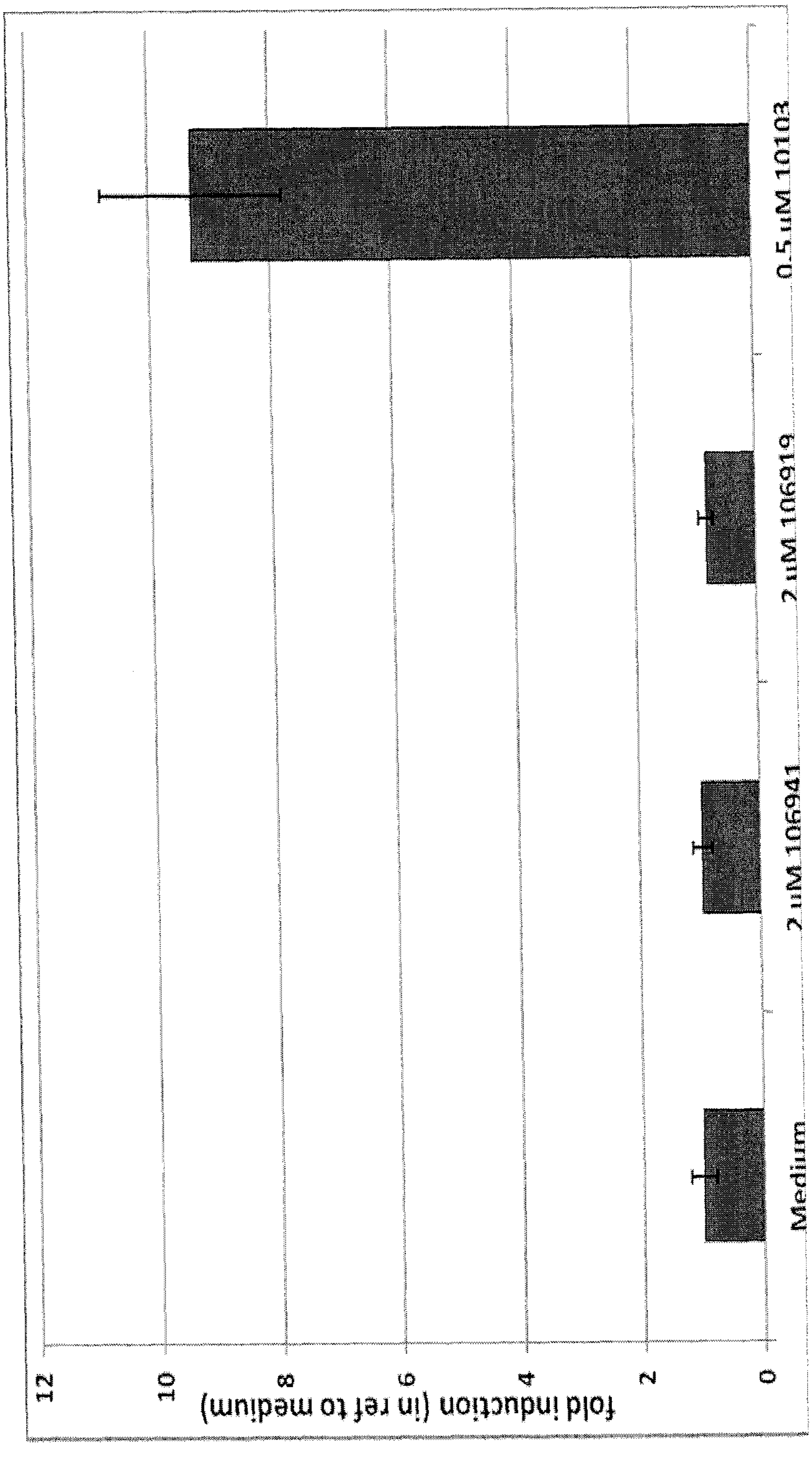
fold induction (in ref to medium)
0
2
4
6
8
10
12
Medium
2 µM 106941
2 µM 106919
0.5 µM 10103

Figure 21B

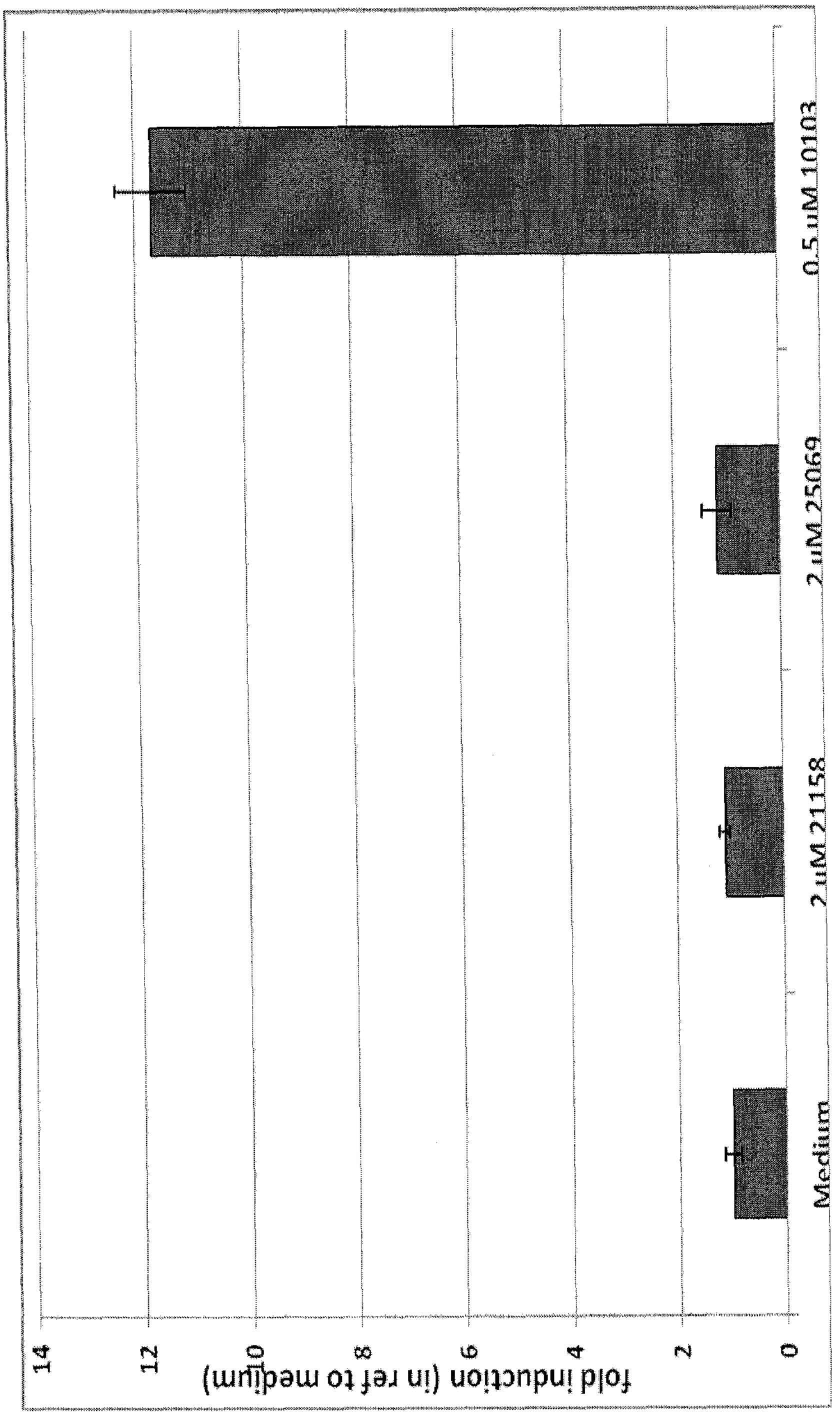
fold induction (in ref to medium)
0
2
4
6
8
10
12
14
Medium
2 µM 21158
2 µM 25069
0.5 µM 10103

Figure 21C

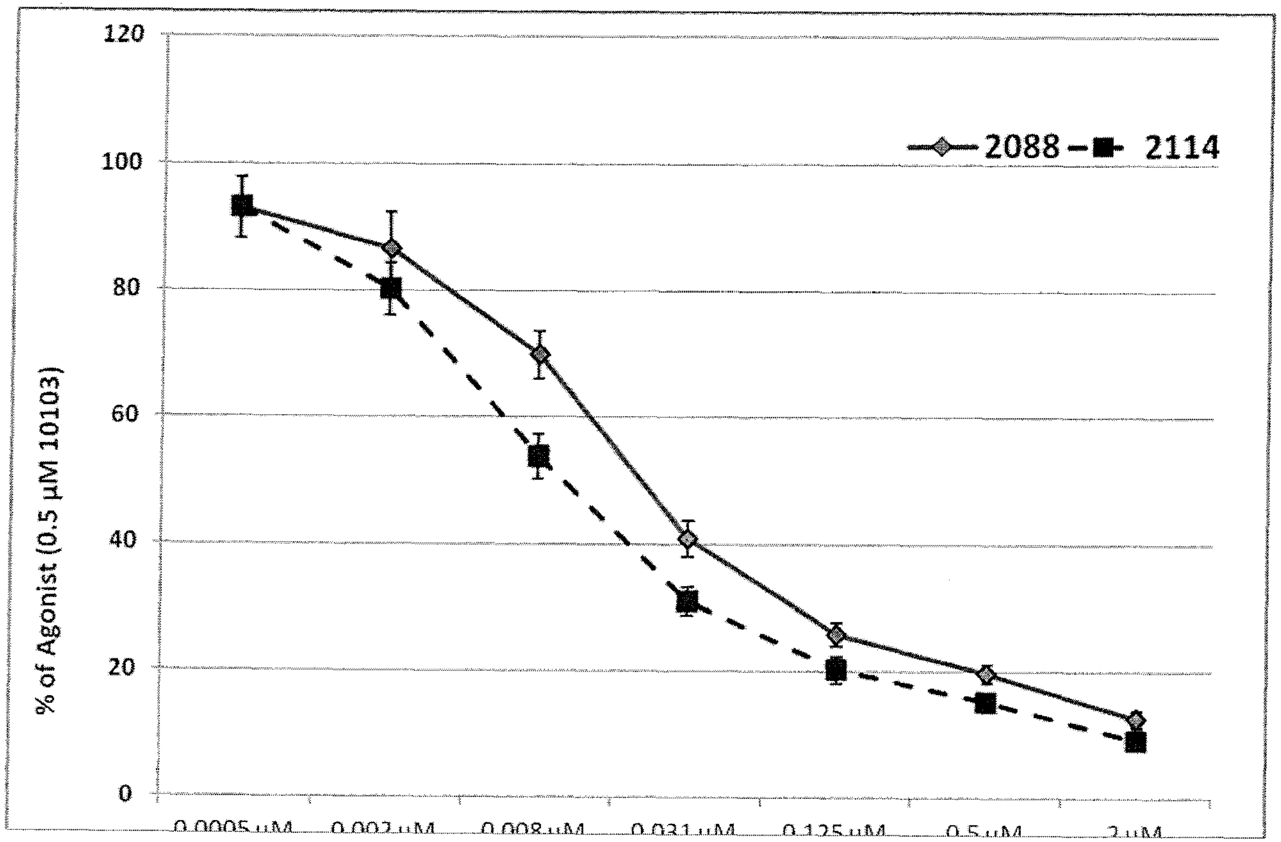


| SEQ ID NO. | CPG Lot | OEX Code | Description |
|---|---|---|---|
| 1 | 2088 | dT*dC*dC*dT*dG*dG*dC*dG*dG*dG*dG*dA*dA*dG*dT | |
| 102 | 2114 | dT*dC*dC*dT*dG*dG*dA*dG*dG*dG*dG*dA*dA*dG*dT | C to A change |

Figure 21D

Europäisches Patentamt
European Patent Office
Office européen des brevets

# EUROPEAN SEARCH REPORT

Application Number
EP 13 15 1106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | US 2005/239733 A1 (JURK MARION [DE] ET AL) 27 October 2005 (2005-10-27)<br>* the whole document *<br>----- | 1-4,9-13<br>1-7,9-13 | INV.<br>C12N15/117<br>A61K31/7088 |
| Y | ROEMMLER F ET AL: "C-modified inhibitory oligonucleotides for treatment of TLR-mediated autoimmune diseases and hyperinflammation", INTERNATIONAL JOURNAL OF MEDICAL MICROBIOLOGY, vol. 302, no. Suppl. 1, HV06, September 2012 (2012-09), page 120, XP002698655,<br>* the whole document *<br>& 64TH ANNUAL MEETING OF THE GERMAN SOCIETY FOR HYGIENE AND MICROBIOLOGY (DGHM); HAMBURG, GERMANY; SEPTEMBER 30 -OCTOBER 03, 2012<br>----- | 1-7,9-13 | ADD.<br>C07H21/00<br>A61P29/00<br>A61P37/02 |
| A | WO 2006/028742 A2 (DYNAVAX TECH CORP [US]; BARRAT FRANCK [US]; COFFMAN ROBERT L [US]) 16 March 2006 (2006-03-16)<br>* the whole document *<br>----- | 1-7,9-13 | TECHNICAL FIELDS SEARCHED (IPC) |
| A,D | EUGEN ULHMANN ET AL: "Recent advances in the development of immunostimulatory oligonucleotides", CURRENT OPINION IN DRUG DISCOVERY AND DEVELOPMENT, vol. 6, no. 2, 1 January 2003 (2003-01-01), pages 204-217, XP009103505, ISSN: 1367-6733<br>* the whole document *<br>----- | 1-7,9-13 | C12N<br>A61K |
| A,D | WO 2011/005942 A2 (IDERA PHARMACEUTICALS INC [US]; YU DONG [US]; BHAGAT LAKSHMI [US]; WAN) 13 January 2011 (2011-01-13)<br>* the whole document *<br>-----<br>-/-- | 1-7,9-13 | |

~~The present search report has been drawn up for all claims~~

1

| Place of search | Date of completion of the search | Examiner |
|---|---|---|
| The Hague | 12 June 2013 | Andres, Serge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 1106

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LENERT PETAR ET AL: "Structural characterization of the inhibitory DNA motif for the type A (D)-CpG-induced cytokine secretion and NK-cell lytic activity in mouse spleen cells", DNA AND CELL BIOLOGY, vol. 22, no. 10, 1 October 2003 (2003-10-01), pages 621-631, XP002422640, ISSN: 1044-5498, DOI: 10.1089/104454903770238094 * the whole document * ----- | 1-7,9-13 | |
| A | ROBBINS MARJORIE ET AL: "2'-O-methyl-modified RNAs act as TLR7 antagonists", MOLECULAR THERAPY, vol. 15, no. 9, 1 September 2007 (2007-09-01), pages 1663-1669, XP002590543, ISSN: 1525-0016, DOI: 10.1038/SJ.MT.6300240 * the whole document * ----- | 1-7,9-13 | |
| | | | TECHNICAL FIELDS SEARCHED (IPC) |

~~The present search report has been drawn up for all claims~~

1

| Place of search | Date of completion of the search | Examiner |
|---|---|---|
| The Hague | 12 June 2013 | Andres, Serge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 13 15 1106

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-7(completely); 9-13(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION
## SHEET B

Application Number
EP 13 15 1106

The Search Division considers that the present European patent application does not comply with the requirements of unity of invention and relates to several inventions or groups of inventions, namely:

1. claims: 1-7(completely); 9-13(partially)

   Inhibitory oligonucleotides having one of formulae (I) to (III), compositions comprising them and their therapeutic uses.

   ---

2. claims: 8(completely); 9-13(partially)

   Inhibitory oligonucleotides having formula (IV), compositions comprising them and their therapeutic uses.

   ---

## ANNEX TO THE EUROPEAN SEARCH REPORT ON EUROPEAN PATENT APPLICATION NO. EP 13 15 1106

This annex lists the patent family members relating to the patent documents cited in the above-mentioned European search report. The members are as contained in the European Patent Office EDP file on
The European Patent Office is in no way liable for these particulars which are merely given for the purpose of information.

12-06-2013

| Patent document cited in search report | Publication date | Patent family member(s) | Publication date |
|---|---|---|---|
| US 2005239733 A1 | 27-10-2005 | NONE | |
| WO 2006028742 A2 | 16-03-2006 | AU 2005282889 A1 | 16-03-2006 |
| | | AU 2012203156 A1 | 21-06-2012 |
| | | CA 2578775 A1 | 16-03-2006 |
| | | CN 101052643 A | 10-10-2007 |
| | | DK 1794174 T3 | 09-07-2012 |
| | | EP 1794174 A2 | 13-06-2007 |
| | | EP 2305693 A1 | 06-04-2011 |
| | | ES 2385657 T3 | 27-07-2012 |
| | | JP 2008514187 A | 08-05-2008 |
| | | JP 2012191939 A | 11-10-2012 |
| | | KR 20070047837 A | 07-05-2007 |
| | | NZ 553581 A | 29-04-2011 |
| | | PL 1794174 T3 | 30-11-2012 |
| | | PT 1794174 E | 16-08-2012 |
| | | SI 1794174 T1 | 28-09-2012 |
| | | US 2007238678 A1 | 11-10-2007 |
| | | WO 2006028742 A2 | 16-03-2006 |
| WO 2011005942 A2 | 13-01-2011 | EP 2451974 A2 | 16-05-2012 |
| | | US 2011009477 A1 | 13-01-2011 |
| | | WO 2011005942 A2 | 13-01-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050239733 A
- WO 2011005942 A


### Non-patent literature cited in the description


- **UHLMANN et al.** *Curr Opin Drug Discov Deve,* 2003, vol. 6, 204-17
- **UHLMANN E et al.** *Chem. Rev.,* 1990, vol. 90, 543
- Protocols for Oligonucleotides and Analogs. *Nucleotides, their derivatives and the synthesis therof*
- **HABERMEHL et al.** Naturstoffchemie. Springer, 2008
- Remington's Pharmaceutical Sciences. Mack Publishing Co, 1990
- **BAUER et al.** *PNAS,* 2001, vol. 98 (16), 9237-42
- **HEIL et al.** *Science,* 2004, vol. 303 (5663), 1529-9
- **JURK et al.** *Eur J. Immunol.,* 2006, vol. 36 (7), 1815-26
- **LUGANINI et al.** *Antimicrob. Agents Chemother.,* 2008, vol. 52, 1111-1120
- **JURK et al.** *Eur. J. Immunol,* 2006, vol. 36 (7), 1815-26
- **MAKINO et al.** *J. Nippon Moed Sch,* 2012, vol. 79, 129-138
- **ROLLER et al.** *J Immunol.,* 2012, vol. 189, 4612-4620